(19) **Europäisches Patentamt**
**European Patent Office**
**Office européen des brevets**

(11) **EP 3 415 649 B1**

(12) **EUROPEAN PATENT SPECIFICATION**

(45) Date of publication and mention
of the grant of the patent:
**03.08.2022 Bulletin 2022/31**

(51) International Patent Classification (IPC):
***C22C 19/05*** (2006.01)

(21) Application number: **17175922.8**

(22) Date of filing: **14.06.2017**

(52) Cooperative Patent Classification (CPC):
**H01B 1/02; C22C 19/05; C22C 19/055;**
**C22C 19/056; C22C 30/00;** C22F 1/16

(54) **A COMPOSITE WIRE**

VERBUNDDRAHT

FIL COMPOSITE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**19.12.2018 Bulletin 2018/51**

(73) Proprietors:
• **Heraeus Deutschland GmbH & Co. KG**
**63450 Hanau (DE)**
• **Saes Smart Materials, Inc.**
**New Hartford, NY 13413 (US)**
• **Heraeus Materials Singapore Pte. Ltd.**
**569881 Singapore (SG)**
• **Heraeus Medical Components, LLC**
**St. Paul, MN 55127 (US)**

(72) Inventors:
• **RICHTER, René**
**64832 Babenhausen (DE)**
• **GEBERT, Dr. Jörg-Martin**
**76227 Karlsruhe (DE)**

• **SPECHT, Heiko**
**63456 Hanau (DE)**
• **SCZERZENIE, Francis E.**
**Lake Pleasant, NY New York 12108 (US)**
• **MANJERI, Radhakrishnan M.**
**New Hartford, NY New York 13413-2250 (US)**
• **YIN, Weimin**
**Ridgefield, CT Connecticut 06877 (US)**
• **GOLAGANI VENKATA KANAKA, Sai Srikanth**
**730586 Singapore (SG)**
• **LARK, Larry**
**Saint Paul, MN Minnesota 55116 (US)**

(74) Representative: **Maiwald Patent- und**
**Rechtsanwaltsgesellschaft mbH**
**Elisenhof**
**Elisenstraße 3**
**80335 München (DE)**

(56) References cited:
**WO-A1-2005/026399 WO-A1-2016/064790**
**US-A1- 2015 099 958**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the invention

The invention generally relates to a composite wire, a coil comprising at least two composite wires, a cable comprising at least three composite wires, a medical device comprising such composite wire, coil and/or cable and a manufacturing method for a composite wire. The composite wire comprises a first part and a second part. The first part is a metallic component. The second part comprises an alloy comprising Cr, Ni, Mo and Co, preferably with tightly controlled levels of impurities.

Background of the invention

Much investigation in recent years has been directed to a search for new high performance alloys, particularly for medical applications where a very high value is placed on reliability and materials are required which exhibit a low failure rate even over a long time period.

Cardiac Pacemakers, Implantable Cardioverter Defibrillation Devices and Cardiac Resynchronisation Devices are applications where reliability is particularly important, especially in terms of resistance to physical fatigue and to chemical corrosion. Invasive surgery is required to implant a pacemaker into the body or remove or replace parts, and it is highly desirable for the individual components of the pacemaker to have a long working life in order to reduce the requirement for surgical intervention. Furthermore, it is desirable for the working life to have a low variance. In a heart pacemaker, one component, which is exposed to a particularly high amount of stress during normal operation is the so called lead which connects the implantable pulse generator to the heart tissue. A flexible lead is required in order to connect the implantable pulse generator to the heart tissue without imposing undue physical stress on the heart and the lead flexes during normal operation, typically repetitively with a frequency on the order of that of a human heart beat. A high resistance to fatigue is therefore required in the lead in order to withstand frequent physical stress over a long period of time. A high resistance of the lead to corrosion is important not only in terms of the lifetime of the component, but also in terms of reducing toxicity to the body.

WO 2005026399 A1 discusses an approach to improving the properties of an alloy by reducing the content of titanium nitride and mixed metal carbonitride.

US 2005/0051243 A1 focuses on alloys with a reduced content of nitrogen.

Implanted medical leads and wires require material that is of sufficient fatigue resistance and electrical conductivity. In many applications, such as those for implant into the brain or where the wire material is in direct contact with the body to electrically shock or stimulate tissue, further increased fatigue resistance and electrical conductivity properties are needed.

Summary of the invention

Hence, there may be a need to provide an improved composite wire, which has in particular improved fatigue resistance and electrical conductivity properties.

The problem of the present invention is solved by the subject-matters of the independent claims, wherein further embodiments are incorporated in the dependent claims. It should be noted that the aspects of the invention described in the following apply also to the composite wire, the coil comprising at least two composite wires, the cable comprising at least three composite wires, the medical device comprising such composite wire, coil and/or cable and the manufacturing method for a composite wire.

According to the present invention, a composite wire is presented. The composite wire comprises a first part and a second part. The first part is a metallic component.

The second part comprises an alloy comprising the following alloy components:

a) Cr in the range from about 10 to about 30 wt. %;
b) Ni in the range from about 20 to about 50 wt. %;
c) Mo in the range from about 2 to about 20 wt. %;
d) Co in the range from about 10 to about 50 wt. %.

The Al content of the Cr, Ni, Mo and Co alloy is less than about 0.01 wt. % and each wt. % is based on the total weight of the alloy.

The wire may be a single strand or rod of metal or may comprise a bundle of such strands or rods. The wire may be configured to bear mechanical loads or electricity and/or telecommunications signals. The wire may be flexible and may be circular in cross-section or square, hexagonal, flattened, rectangular or the like.

A composite wire may comprise at least two different materials.

The first part may be a metal or an alloy and may provide or enhance an electrical conductivity of the composite wire.

The second part comprises above mentioned alloy, which is a Cr, Ni, Mo and Co alloy. The Cr, Ni, Mo and Co alloy may be cleaner with less impurities and less and smaller inclusions which may lead to an improved resistance to physical fatigue. The Cr, Ni, Mo and Co alloy may therefore have an improved resistance to physical fatigue, a high corrosion resistance and/or the ability to be drawn into a thin wire. In an example, the Cr, Ni, Mo and Co components are major constituents of the Cr, Ni, Mo and Co alloy with at least about 95 wt. % of the alloy being Cr, Ni, Mo and Co. Details in view of the alloy are provided further below.

The composite wire according to the invention combines a first metallic part with a second part of a high purity Cr, Ni, Mo and Co alloy. The first metallic part provides electrical conductivity and the Cr, Ni, Mo and Co alloy and in particular, its high purity, provides an excellent fatigue resistance. As a result, the composite wire meets at the same time electrical conductivity and fatigue resistance requirements for use in highly sensitive implanted applications such as the human brain or other applications where direct and prolonged contact with the body is required, such as in sensing and stimulation applications.

In an example, the first part at least partially surrounds the second part when seen in a cross section. The first part may also essentially or completely surround the second part when seen in a cross section. The first part may be a clad, cover, coating or the like. In an example, the first part is biocompatible. Consequently, the first part may provide a biocompatible clad to the composite wire and thereby greatly improve its biocompatibility. The first part may also or additionally be corrosion resistant, which not only avoids corrosion, but also the occurrence of corrosion products and their cytotoxicity. As a result, also the biostability of the composite wire may be greatly improved. Increased biocompatibility and/or biostability may be considerably important for implants into the brain or into other locations where the composite wire is in direct and prolonged contact with the body to e.g. electrically shock or stimulate tissue.

In an example, the first part comprises at least one of a group of Platinum, a Platinum based alloy, a Platinum-Iridium alloy, a Platinum-Tungsten alloy, Gold, a Gold alloy, Tantalum, Titanium, a Titanium-Molybdenum alloy, a Titanium Aluminum Vanadium alloy and the like. In an example, the first part comprises a Platinum-Iridium alloy with about 70-90 wt. % Platinum and 10-30 wt. % Iridium. Such composition and Platinum and Platinum based alloys in general may enhance the biocompatibility and/or biostability of the composite wire for use as e.g. a chronic implant.

In an example, the composite wire further comprises a core part, which is at least partially surrounded by the second part when seen in a cross section. The core part may also be essentially or completely surrounded by the second part when seen in a cross section. In an example, the core part is a metallic component. In an example, the core part is electrically conductive. In an example, the core part comprises at least one of a group of Silver, Platinum and the like. The core part may further improve the electrical properties and in particular the electrical conductivity of the composite wire. A core content of a composite wire may be in the range from 15 to 50 wt. %, preferably in the range from 17.5 to 45.7 wt. %, and more preferably in the range from 28.7 to 37.7 wt. % based on the total weight of the wire.

As described above, the first part may form an outer shell for the second part comprising the Cr, Ni, Mo and Co alloy. As described below, the second part comprising the Cr, Ni, Mo and Co alloy forms an outer shell for the first part.

In another example, the second part at least partially surrounds the first part when seen in a cross section. The second part may also essentially or completely surround the first part when seen in a cross section. In an example, the first part is a filling material and comprises at least one of a group of Platinum, Tantalum, Gold, Copper, Silver and alloys thereof. In another example, the first part is not pure Silver. It may be a Silver alloy as e.g. AgMg1. In particular Platinum and Platinum based alloys may enhance biocompatibility of the composite wire. Filling materials such as Ta, Pt, Au, Cu provide or increase radiopacity, electrical conductivity and/or a melting temperature (e.g. AgMg-core compared to pure Ag).

In an example, a filling degree of the filling material or first part relative to the Cr, Ni, Mo and Co alloy or second part is in the range from 15 % to 41 %, preferably in the range from 20 % to 35 %, and more preferably in the range from 23 % to 33 % when seen in a cross section.

In an example, the composite wire further comprises a circumference part, which at least partially surrounds the second part when seen in a cross section. The circumference part may also essentially or completely surround the second part when seen in a cross section. In an example, the circumference part is biocompatible. In an example, the circumference part comprises at least one of a group of Platinum, a Platinum based alloy, a Platinum-Iridium alloy, a Platinum-Tungsten alloy, Gold, a Gold alloy, Tantalum, Titanium, a Titanium-Molybdenum alloy, a Titanium Aluminum Vanadium alloy and the like. In particular Platinum and Platinum based alloys may enhance biocompatibility of the composite wire.

In an example, a diameter of the composite wire is in a range of 5 to 500 $\mu$m, preferably in the range of 10 to 250 $\mu$m, and more preferably in the range of 15 to 35 $\mu$m. The diameter of the composite wire used in medical applications could range from 10,2 to 508 $\mu$m ( 0.0004" to 0.020"), more commonly from 17,8 to 254 $\mu$m (0.0007" to 0.010").

In an example, the composite wire further comprises a coating as outermost part at least partially surrounding all other parts. The coating may also essentially or completely surround all other parts. The coating may be applied independent of the structure and materials of the composite wire. In other words, the coating may be applied on the first part, the second part or the circumference part, whichever part is the outermost part of the composite wire. The coating may also be applied on the composite wire in straight or coiled condition, on several composite wires lateron forming a cable or strand, or the cable or the strand itself, which comprises several composite wires.

In an example, the coating provides or enhances electrical insulation, electrical conductivity, mechanical properties, lubricity, biocompatibility and/or biostability of the composite wire. In an example, the coating comprises a polymer or other organic based coating. The coating may be a solvent based and cured polymer. The coating may be soluble in organic solvents, water and/or mixtures thereof. The coating may comprise at least one of a group of ETFE, PFA, Polyimide, Polyamide, PTFE, Polyurethane, PEEK, Parylene or the like. The coating may comprise Iridium Oxide, Titanium Nitride or the like.

In an example, the coating provides or enhances electrical insulation for e.g. conductor applications. The coating may then comprise at least a non-conductive polymer, a ceramic and/or a cermet. The coating may also comprise at least one of a group of diamond-like carbon, carbon nanotube, graphite, other carbon-based coatings or the like.

In another example, the coating provides or enhances electrical conductivity and/or reduces resistance and/or impedance. The coating may then comprise at least a conductive polymer. In an example, the coating comprises at least one of a group of PEDOT, PEDOT:PSS or the like.

In an example, the coating provides or enhances (long-term) biostability in the body and/or charge capacitance for improved contact to tissue and/or other biological properties. The coating may then comprise at least one of a group of enzymes, antimicrobials, steroids, other drugs or biological active substances or the like. The coating may also reduce body tissue inflammatory response or damage from the implanted composite wire.

A thickness of the coating may range from a nanometer scale to millimeter scale depending on the application. Biological and conductive coatings may be on a smaller nano or micro scale, whereas insulative coatings may range from 1 $\mu$m to 100 $\mu$m. The thickness of the coating may range from 5 to 50 $\mu$m, preferably from 10 to 20 $\mu$m.

The coating materials can be applied continuously by e.g. thermoplastic polymer extrusion, dip coating or enameling processes.

The composite wires as described above can be formed into coils or cables for use in medical lead applications such as pacemaker leads and neurostimulation leads. In the coil form, the coil can comprise a single wire or multiple wires. The multiple wires can comprise a single conductor path or be electrically insulated to comprise multiple separate electrical paths. In the cable configuration, the cable may comprise multiple wires twisted together in various configurations in order to improve flexibility of the overall strand. Likewise, the cable can comprise a single electrical path or multiple groups of wires, each electrically isolated by insulator coatings. The cables may then be used in a straight configuration or can be further wound into a coil form similar as described above.

According to the present invention, a coil comprising at least two composite wires as described above is presented, wherein the at least two composite wires are wound or coiled together.

Possible coil configurations (but not limited to) are single or multi-filar coils (up to 8 wires forming one coil). They can be tight wound with no gap between coiled wires or open wound with a gap of e.g. 5 to 50 $\mu$m between wires. The coils may have outer diameters of 50 $\mu$m to 3 mm. In an example, the coil comprises 1 to 16 composite wires wound in 1 to 3 concentric sub coils. Preferably, 1 to 8 composite wires are wound in one individual coil. In an example, at least some of the composite wires are electrically insulated from each other.

In an example, a diameter of a composite wire in a coil is in a range of 50 to 250 $\mu$m, preferably in a range of 100 to 200 $\mu$m.

According to the present invention, also a cable comprising at least three composite wires as described above is presented, wherein the at least three composite wires are stranded together.

Possible cable configurations (but not limited to) are 1x3, 3x3, 3x3x3, 3x7, 7x3, 3x19, 19x3, 19x7, 7x19, 7x7x7, 7x7, 1x7, 1×19 and the like. In an example, the cable comprises 3 to 361 composite wires stranded together. In an example, the at least some of the composite wires are electrically insulated from each other. Preferably, 3 to 133 wires and more preferably 7 to 49 wires are stranded together into one cable.

In an example, a diameter of a composite wire in a cable is in a range of 5 to 50 $\mu$m, preferably in a range of 15 to 35 $\mu$m.

The wires, coils and cables may be coated with polymers to provide electrical insulation, and used in this form or can be then stranded into multi-conductor cables or coiled into multi-conductor coils. The wires, coils or cables may be used as conductor paths in medical device products. Such conductors paths may be used in leads for sensing or delivering cardiac therapies such as pacing, defibrillating, or cardiac resynchronization, or in leads for sensing or delivering stimulation to nerves or neural tissue in areas such as deep brain, spinal cord, vagus nerve, and peripheral nerves.

According to the present invention, a medical device comprising at least one of the group of a composite wire, a coil and a cable as described above is presented, wherein the at least one composite wire, coil and/or cable is used as a lead. In an example, the lead is configured for at least one of a group of: pacing, defibrillating, cardiac rhythm management, resynchronization and/or stimulation to nerves or neural tissue in deep brain, spinal cord, vagus nerve or a peripheral nerve and the like. In an example, the medical device may be a pacemaker, an implantable cardioverter defibrillator, a cardiac resyncronisation device, a neuromodulation device, a cochlea implant or any other implantable stimulation device comprising a composite wire as described above as a lead.

The lead of the medical device may comprise several composite wires (e.g. with a Ta core and a Pt clad on the outside), preferably grouped into two or more cables, each cable comprising two or more composite wires. In one embodiment, the cables have a thickness in the range from 0.05 to about 0.5 mm, preferably in the range from about 0.1 to 0.4 mm.

According to the present invention, a manufacturing method for a composite wire is presented. The manufacturing method for a composite wire comprises the following steps, not necessarily in this order:

- providing a first part and a second part concentric to each other when seen in a cross section, and
- joining the first part and the second part together.

The first part is a metallic component. The second part comprises an alloy comprising the following alloy components:

   a) Cr in the range from about 10 to about 30 wt. %;
   b) Ni in the range from about 20 to about 50 wt. %;
   c) Mo in the range from about 2 to about 20 wt. %;
   d) Co in the range from about 10 to about 50 wt. %.

The Al content of the Cr, Ni, Mo and Co alloy is less than about 0.01 wt. % and each wt. % is based on the total weight of the alloy.

In an example, the composite wire may be produced in that the material undergoes a joining process into a single cylinder or rod of material that comprises the at least two concentric parts and materials. Such process can involve joining rods and cylinders of material into a single cylinder comprising the desired materials and cross-section ratios of the materials. A single cylinder may or may not be further jacketed for protection, and then extruded, swaged, or drawn through a series of dies to reduce its diameter. This process may be repeated with multiple reduction and heat treatment steps in order to reduce the composite wire diameter to desired diameter and mechanical properties. Of course, also a third or even more materials may be included as three or more concentric parts and materials in the above described joining process into a single cylinder or rod of material.

In an example, the Cr, Ni, Mo and Co alloy is molten, forged and rolled to bar sizes of 25,4-38,1 mm (~1-1.5",), surface peeled, shaved or ground, and gun-drilled to create a hollow for tube drawing. A tube processing may comprise multiple steps of drawing, cleaning and annealing to create smaller tube outer diameters of 2,5-7,6 mm (0.1 - 0.3')

. The tube may be filled with a core material in form of a rod slightly smaller than the outer diameter of the tube. The filled tube may be drawn through e.g. diamond dies to close a gap between a core material and an outer oy material. This composite wire can then be used as second core to fill an e.g. Pt or Pt-alloy tube which forms an outermost layer and coating of a coated composite wire. The coated composite wire can then be drawn to smaller sizes using e.g. a mineral drawing oil and diamond dies. Down to small sizes, the coated composite wire may have to be (in-line) annealed one or multiple times to soften the material for further drawing.

The Cr, Ni, Mo and Co alloy may be an alloy, which has improved resistance to physical fatigue, a high corrosion resistance, and/or which can be drawn into a thin wire, preferably less than about 50 $\mu$m. The wire according to the invention may be a wire having comparable tensile properties to known wires, but for which the proportion of outlying failures in fatigue resistance is reduced.

A contribution to achieving at least one of the above described objects is made by the following Cr, Ni, Mo and Co alloy (in the following "alloy").

   |1| An alloy comprising the following alloy components:

      a) Cr in the range from about 10 to about 30 wt. %, preferably in the range from about 15 to about 25 wt. %, more preferably in the range from about 19 to about 21 wt. %;
      b) Ni in the range from about 20 to about 50 wt. %, preferably in the range from about 30 to about 45 wt. %, more preferably in the range from about 33 to about 37 wt. %;
      c) Mo in the range from about 2 to about 20 wt. %, preferably in the range from about 5 to about 15 wt. %, more preferably in the range from about 9 to about 10.5 wt. %;
      d) Co in the range from about 10 to about 50 wt. %, preferably in the range from about 20 to about 40 wt. %, more preferably in the range from about 33 to about 37 wt. %;

wherein the Al content of the alloy is less than about 0.01 wt. %, preferably less than about 0.005 wt. %, more preferably less than about 0.001 wt. %;

wherein each wt. % is based on the total weight of the alloy.

|2| The alloy according to embodiment 111, wherein the content of Mg is less than about 0.005 wt. %, preferably less than about 0.0001 wt. %, more preferably less than about 0.00001 wt. %, based on the total weight of the alloy.

|3| The alloy according to embodiment |1| or |2|, wherein the content of Ca is less than about 0.005 wt. %, preferably less than about 0.0001 wt. % more preferably less than about 0.00001 wt. %, based on the total weight of the alloy.

|4| The alloy according to any of the preceding embodiments, wherein the content of Ce is less than about 0.005 wt. %, preferably less than about 0.0001 wt. % more preferably less than about 0.00001 wt. %, based on the total weight of the alloy.

|5| The alloy according to any of the preceding embodiments, wherein the content of Ti is less than about 0.1 wt. %, preferably less than about 0.01 wt. % more preferably less than about 0.001 wt. %, further more preferably less than about 0.0005 wt. %, based on the total weight of the alloy.

|6| The alloy according to any of the preceding embodiments, wherein the content of Fe is in the range from about 0.0001 to about 1 wt. %, preferably in the range from about 0.0005 to about 0.1 wt. %, more preferably in the range from about 0.001 to about 0.05 wt. %, based on the total weight of the alloy.

|7| The alloy according to any of the preceding embodiments, wherein at least one of the following is satisfied:

a) The content of C in the alloy is less than about 0.1 wt. %, preferably less than about 0.08 wt. % more preferably less than about 0.05 wt. %

b) The content of B in the alloy is less than about 0.01 wt. %, preferably less than about 0.001 wt%, more preferably less than about 0.0002 wt. %;

c) The content of P in the alloy is less than about 0.01 wt. %, preferably less than about 0.005 wt. %, more preferably less than about 0.001 wt. %, further more preferably less than about 0.0005 wt. %;

d) The content of S in the alloy is less than about 0.005 wt. %, preferably less than about 0.003 wt. %, more preferably less than about 0.002 wt. %, further more preferably less than about 0.0008 wt. %;

each wt. % being based on the total weight of the alloy. In preferred aspects of this embodiment, the combination of the above criteria which are satisfied is selected from the group consisting of: a), b), c), d), a)+b), a)+c), a)+d), b)+c), b)+d), c)+d), a)+b)+c), a)+b)+d), a)+c)+d), b)+c)+d) and a)+b)+c)+d).

|8| The alloy according to any of the preceding embodiments, wherein at least one of the following is satisfied:

a) The content of Mn in the alloy is less than about 0.05 wt. %, preferably less than about 0.005 wt. %, more preferably less than about 0.001 wt. %;

b) The content of Si in the alloy is less than about 0.05 wt. %, preferably less than about 0.03 wt. %, more preferably less than about 0.02 wt. %;

each wt. % being based on the total weight of the alloy. In preferred aspects of this embodiment, the combination of the above criteria which are satisfied is selected from the group consisting of: a), b), a)+b).

|9| The alloy according to any of the preceding embodiments, wherein at least one of the following is satisfied:

a) The content of O in the alloy is in the range from about 0.0001 to about 0.05 wt. %, preferably in the range from about 0.0001 to about 0.03 wt. %, more preferably in the range from about 0.0001 to about 0.01 wt. %;

b) The content of N in the alloy is in the range from about 0.0001 to about 0.01 wt. %, preferably in the range from about 0.0001 to about 0.008 wt. %, more preferably in the range from about 0.0001 to about 0.005 wt. %;

each wt. % being based on the total weight of the alloy. In preferred aspects of this embodiment, the combination of the above criteria which are satisfied is selected from the group consisting of: a), b), a)+b).

|10| The alloy according to any of the preceding embodiments, wherein at least one of the following is satisfied:

a) The alloy contains less than about 0.01 wt. %, preferably less than about 0.005 wt. %, more preferably less than about 0.001 wt. %, O in the form of a magnesium oxide;
b) The alloy contains less than about 0.01 wt. %, preferably less than about 0.005 wt. %, more preferably less than about 0.001 wt. %, O in the form of an aluminium oxide;
c) The alloy contains less than about 0.01 wt. %, preferably less than about 0.005 wt. %, more preferably less than about 0.001 wt. %, O in the form of a cerium oxide.
d) The alloy contains less than about 0.01 wt. %, preferably less than about 0.005 wt. %, more preferably less than about 0.001 wt. %, O in the form of a calcium oxide.
e) The alloy contains less than about 0.01 wt. %, preferably less than about 0.005 wt. %, more preferably less than about 0.001 wt. %, O in the form of a chromium oxide.
In preferred aspects of this embodiment, the combination of the above criteria which are satisfied is selected from the group consisting of: a), b), c), d), a)+b), a)+c), a)+d), b)+c), b)+d), c)+d), a)+b)+c), a)+b)+d), a)+c)+d), b)+c)+d), a)+b)+c)+d), e), a)+e), b)+e), c)+e), d)+e), a)+b)+e), a)+c)+e), a)+d)+e), b)+c)+e), b)+d)+e), c)+d)+e), a)+b)+c)+e), a)+b)+d)+e), a)+c)+d)+e), b)+c)+d)+e) and a)+b)+c)+d)+e).

|11| A process for the preparation of an alloy comprising the following preparation steps:

a) Provision of a mixture comprising the following components:

i. Cr in the range from about 10 to about 30 wt. %, preferably in the range from about 15 to about 25 wt. %, more preferably in the range from about 19 to about 21 wt. %;
ii. Ni in the range from about 20 to about 50 wt. %, preferably in the range from about 25 to about 40 wt. %, more preferably in the range from about 33 to about 37 wt. %;
iii. Mo in the range from about 2 to about 20 wt. %, preferably in the range from about 5 to about 15 wt. %, more preferably in the range from about 9 to about 10.5 wt. %;
iv. Co in the range from about 10 to about 50 wt. %, preferably in the range from about 20 to about 40 wt. %, more preferably in the range from about 33 to about 37 wt. %.

wherein each wt. % is based on the total weight of the mixture prepared for melting;
b) Melting the mixture in a vacuum induction melting step in order to obtain a first melt, in one aspect of this embodiment, one or more further vacuum induction melting steps are carried out;
c) Solidifying the first melt in order to obtain a first solid;
d) Melting the first solid in a vacuum arc melting step in order to obtain a further melt;
e) Solidifying the further melt in order to obtain a further solid.

|12| The process according to embodiment |11|, wherein pressure in step b) is below about 100 hPa (0.1 bar), preferably below about 50 hPa (0.05 bar), more preferably below about 10 hPa (0.01 bar).

|13| The process according to embodiment |11| or |12|, wherein the leak rate in step b) is below about 100 hPa / min (0.1 bar / min), preferably below about 50 hPa/min (0.05 bar/min), more preferably below about 10 hPa / min (0.01 bar/min).

|14| The process according to any of the embodiments |11| to |13|, wherein the pressure in step d) is below about 50 hPa (0.05 bar), preferably below about 10 hPa (0.01 bar), more preferably below about 5 hPa (0.005 bar).

|15| The process according to any of the embodiments |11| to |14|, wherein the leak rate in step d) is below about 50 hPa /min (0.05 bar /min), preferably less than about 10 hPa /min (0.01 bar /min), more preferably less than about 5 hPa /min (0.005 bar /min).

|16| The process according to any of the embodiments |11| to |15|, further comprising a homogenisation step carried out at a temperature in the range from about 900 to about 1300 °C, preferably in the range from about 1000 to about 1250 °C, more preferably in the range from about 1100 to about 1225 °C.

|17| The process according to any of the embodiments |11| to |16|, further comprising a cogging step carried out at a temperature in the range from about 900 to about 1300 °C, preferably in the range from about 1000 to about 1250 °C, more preferably in the range from about 1100 to about 1225 °C.

|18| The process according to any of the embodiments |11| to |17|, further comprising a finish roll step carried out at a temperature in the range from about 900 to about 1300 °C, preferably in the range from about 1000 to about 1250 °C, more preferably in the range from about 1100 to about 1225 °C.

|19| The process according to any of the embodiments |11| to |18|, further comprising a straightening step. In one aspect of this embodiment, the straightening is a hot straightening, preferably carried out at a temperature in the range from about 900 to about 1200 °C, preferably in the range from about 950 to about 1100 °C, more preferably in the range from about 1000 to about 1075 °C. In one aspect of this embodiment, the straightening is a cold straightening, preferably carried out at ambient temperature, preferably at a temperature in the range from about 10 to about 100 °C, more preferably in the range from about 15 to about 80 °C, most preferably in the range from about 20 to about 50 °C.

|20| An alloy obtainable by a process according to any of the embodiments |11| to |19|.

|21| An electrical wire comprising an alloy according to any of the embodiments |1| to |10| or |20|.

|22| A medical device comprising a wire according to embodiment |21|.

|23| A pacemaker, an implantable cardioverter defibrillator, a cardiac resyncronisation device, a neuromodulation device, a cochlea implant or any other implantable stimulation device comprising a wire according to embodiment |21|.

Alloy

The Cr, Ni, Mo and Co alloy comprise two or more elements, preferably as a solid mixture, preferably with an enthalpy of mixing of the constituent elements of less than about 10 KJ/mol, preferably less than about 5 KJ/mol, more preferably less than about 1 KJ/mol. The Cr, Ni, Mo and Co alloy comprise Cr, Ni, Mo and Co as major constituents, preferably with at least about 95 wt. %, more preferably at least about 99 wt. %, further more preferably at least about 99.9 wt. %, more preferably at least about 99.95 wt. % of the alloy being Cr, Ni, Mo and Co.
A composition of the Cr, Ni, Mo and Co alloy is preferred which improves favourable properties of the alloy, in particular resistance to fatigue and/or corrosion resistance, preferably both.
It is preferred for the properties of the alloy to be improved by limiting the content of impurities or limiting the content of a combination of different impurities, preferably according the embodiments of the invention.
It is preferred that there be a low, preferably zero concentration of inclusions in the alloy. This is preferably achieved by limiting the content of impurities. In one embodiment it is preferred that the alloy contain less than about 0.01 %, preferably less than about 0.005 %, more preferably less than about 0.001 % inclusions. The % of inclusions is preferably determined using the microscopic inspection method given in the test methods. Content of inclusions as % is there determined as the proportion of the cross sectional area of the sample surface made up of inclusions. In some instances, the alloy comprises a low, preferably a zero concentration of inorganic non-metallic solid inclusions, more preferably of inorganic oxide inclusions. Inorganic oxides in this context can refer to metal oxides, non-metal oxides and metalloid-oxides. In some cases the the alloy comprises a low, preferably a zero concentration of inclusions comprising one or more selected from the group consisting of: Si, Al, Ti, Zr and B; preferably selected form the group consisting of: Si Ti, and Al.
In one embodiment, one or more treating material(s) is/are contacted with the mixture of the process in order to remove oxygen from the mixture of the process, preferably by incorporation of the oxygen into a dross and removal of the dross. Preferred treating materials in this context comprise one or more selected from the list consisting of: Al, Mg, Ca and Ce; preferably in the form of an element and/or in the form of an alloy, wherein the alloy preferably contains a further metal being selected from group consisting of Cr, Ni, Mo and Co or at least two thereof, preferably Ni.
In order to achieve the preferred concentrations of constituents of the alloy, described above in the embodiments, the skilled person may vary the proportions of starting materials employed in the preparation process. The proportions of the starting materials might not be equal to the proportions of constituents of the product, due to net loss or gain during the preparation process.

Process for preparation of the alloy

The process for the preparation of the alloy preferably comprises the following steps:

a) A vacuum induction melting step;
b) A vacuum arc melting step.

In one embodiment of the invention, the process comprises two or more vacuum induction melting steps. In another embodiment of the invention, the process comprises two or more vacuum melting steps. In another embodiment of the invention, the process comprises two or more vacuum induction melting steps and two or more vacuum arc melting steps.

In preferred embodiments of the invention, the process further comprises one or more of the following steps:

c) An electro-slag melting step
d) A homogenisation step
e) A cogging step
f) A finish roll step
g) A straightening step

In preferred embodiments, the process comprises a combination of the above steps selected from the list consisting of: c), d), e), f), g), c)+d), c)+e), c)+f), c)+g), d)+e), d)+f), d)+g), e)+f), e)+g), f)+g), c)+d)+e), c)+d)+f), c)+d)+g), c)+e)+f), c)+e)+g), c)+f)+g), d)+e)+f), d)+e)+g), d)+f)+g), e)+f)+g), d)+e)+f)+g), c)+e)+f)+g), c)+d)+f)+g), c)+d)+e)+g), c)+d)+e)+f) and c)+d)+e)+f)+g).

In one embodiment of the invention, one or more of the steps c)-g) is carried out two or more times.

In preferred vacuum induction melting steps, a material is heated by inducing an electric current in the material, preferably by electromagnetic induction. The pressure in the vacuum induction melting step is preferably below about 0.1 hPa (0.1 mbar), more preferably below about 0.01 hPa (0.01 mbar), most preferably below about 0.001 hPa (0.001 mbar). The vacuum induction melt step is preferably carried out in an oven, preferably with a low leak rate, preferably below about 0.1 hPa * 1 / s (0.1 mbar * 1 / s), more preferably below about 0.01 hPa * 1 / s (0.01 mbar * 1 / s), most preferably below about 0.001 hPa * 1 / s (0.001 mbar * 1 / s). The leak rate is preferably tested before the vacuum induction melting step by evacuating the oven, closing the valves of the oven, and measuring the rate of increase of pressure in the oven.

In one embodiment of the invention, the vacuum induction melting step is carried out in an inert atmosphere, preferably argon, preferably an atmosphere comprising at least about 90 wt. %, more preferably at least about 99 wt. %, most preferably at least about 99.9 wt. % of inert gas, preferably argon. In one aspect of this embodiment, the oven is evacuated and inert gas, preferably argon, introduced into the oven before melting. In one aspect of this embodiment, the pressure in the vacuum induction melting step is in the range from about 1 to about 200 mbar, preferably in the arrange from about 10 to about 150 mbar, most preferably in the range from about 20 to about 100 mbar.

In preferred vacuum arc melting steps, a material is heated by passing an electrical current through the material, preferably with an electrical power in the range from about 300 to about 1200 W/kg, more preferably in the range from about 400 to about 1000 W/kg, most preferably in the range from about 450 to about 900 W/kg, based on the mass of material heated. The pressure in the vacuum arc melting step is preferably below about 0.1 hPa (0.1 mbar), more preferably below about 0.01 hPa (0.01 mbar), most preferably below about 0.001 hPa (0.001 mbar). The vacuum arc melt step is preferably carried out in an oven, preferably with a low leak rate, preferably below about 0.1 hPa * 1 / s (0.1 mbar * 1 / s), more preferably below about 0.05 hPa * 1 / s (0.05 mbar * 1 / s), most preferably below about 0.01 * 1 / s (0.01 mbar * 1 / s).

The leak rate is preferably tested before the vacuum arc melting step by evacuating the oven, closing the valves of the oven, and measuring the rate of increase of pressure in the oven. In one embodiment of the invention, the vacuum arc melting step is carried out in an inert atmosphere, preferably argon, preferably an atmosphere comprising at least about 90 wt. %, more preferably at least about 99 wt. %, most preferably at least about 99.9 wt. % of inert gas, preferably argon. In one aspect of this embodiment, the oven is evacuated and inert gas, preferably argon, introduced into the oven before melting. In one aspect of this embodiment, the pressure in the vacuum arc melting step is in the range from 1 hPa to 200 hPa (0.001 to 0.2 bar), preferably in the range from about 10 hPa to 150 hPa (0.01 to 0.15 bar), most preferably in the range from 50 hPa to 100 hPa (0.05 to 0.1 bar).

Homogenisation steps according to the invention preferably allow reduction of inhomogeneity in a material, preferably by heating. In preferred homogenisation steps according to the invention, a material is heated to a temperature which is below its melting temperature, preferably below its incipient melting temperature. It is preferred that the material be homogenised for a duration in the range from about 10 min. to about 20 hours, more preferably in the range from about 3 hours to about 10 hours, most preferably in the range from about 5 hours to about 8 hours. Homogenisation is preferably carried out in a vacuum or in a gaseous atmosphere, preferably in a gaseous atmosphere. It is preferred that the homogenisation step be carried out close to atmospheric pressure, preferably in the range from 500 hPa to 1500 hPa (0.5 to 1.5 bar), more preferably in the range from 800 hPa to 1200 hPa (0.8 to 1.2 bar), most preferably in the range from 900 hPa to 1100 hPa (0.9 to 1.1 bar). In one preferred embodiment, the homogenisation step is carried out in air.

In preferred cogging steps according to the invention, the porosity or grain size or both of a material are reduced, preferably at elevated temperatures, preferably below the melting point of the material, preferably with the application of compressive force. Compressive forces may be applied locally or in a delocalised manner, preferably by one or more selected from the group consisting of: rolling, pressing, beating and turning. Where the material to be cogged has a mass below about 10 kg, preferably below about 8 kg, more preferably below about 5 kg, rolling is preferred. Where the material to be cogged has a mass above about 10 kg, preferably above about 20 kg, more preferably above about 30 kg, beating or turning is preferred. It is preferred that the smallest dimension of the material is reduced during the cogging process. Preferred finish roll steps according to the invention reduce the smallest dimension of the material, preferably by passing the material through one or more pairs of rolls, preferably below the melting point of the material, more preferably below its incipient melting point. In one embodiment, the finish roll step reduces the porosity or grain size of the material, preferably both. Straightening preferably reduces the physical curvature of the material, preferably so as to facilitate further grinding or machining steps. Straightening is preferably carried out by applying compressive force. The straightening step is preferably carried out below the melting point of the material, more preferably below its incipient melting point. In one embodiment, the process comprises a hot straightening step. In one embodiment, the process comprises a cold straightening step, preferably carried out at around ambient temperature. Cold straightening is preferably carried out at a temperature in the range from about 10 to about 100 °C, more preferably in the range from about 15 to about 80 °C, most preferably in the range from about 20 to about 50 °C.

Leads, wires and Medical Devices

In this text, reference is made variously to a coated or cladded wire, which comprises a wire core and a shell. The shell might be coated or cladded onto the core wire.

A preferred lead according to the invention comprises at least one proximal connector, at least one distal electrode and a flexible elongated conductor that is electrically connecting the electrode(s) to the connector(s). Preferably the elongated conductor is a coiled wire or a cable and comprises the alloy according to the invention.

A contribution to achieving at least one of the above mentioned objects is made by a wire comprising an alloy according to the invention, preferably having a thickness in the range from about 10 to about 50 $\mu$m, preferably in the range from about 15 to about 35 $\mu$m. In one embodiment, the wire further comprises silver metal.

In one embodiment, the lead comprises a silver core and an alloy according to the invention, preferably present as a shell surrounding the silver core.

A contribution to achieving at least one of the above mentioned objects is made by a lead comprising one or more wires according to the invention, preferably grouped into two or more cables, each cable comprising two or more wires according to the invention. In one embodiment, the cables have a thickness in the range from about 0.05 to about 0.5 mm, preferably in the range from about 0.1 to 0.4 mm.

A contribution to achieving at least one of the above mentioned problems is made by a medical device, preferably a pacemaker, comprising a lead according to the invention. A preferred pacemaker comprises:

- An implantable pulse generator;

- One or more leads according to the invention.

In one embodiment, the pacemaker comprises one or more pulsers.

In one embodiment, the pacemaker comprises one or more energy cells, preferably one or more electrical cells.

A process for the preparation of a wire comprises the steps:

a) Providing a tube of alloy according to the invention;
b) At least partially filling the tube with Ag to obtain a composite;
c) One or more drawing steps to reduce the diameter of the composite;
d) Optionally one or more annealing steps to soften the composite and facilitate drawing.

In one embodiment of the invention, the Ag content of the wire obtainable by the process is in the range from about 15 to about 50 wt. %, preferably in the range from about 17.5 to about 45.7 wt. %, more preferably in the range from about 28.7 to about 37.7 wt. %, based on the total weight of the wire.

In one embodiment, the diameter of the wire obtainable by the process is in the range from about 5 to about 50 $\mu$m, preferably in the range from about 15 to about 35 $\mu$m.

In one embodiment, the filling degree of silver in the wire obtainable by the process is in the range from about 15 % to about 41 %, preferably in the range from about 20 % to about 35 %, more preferably in the range from about 23 % to about 33 %.

It shall be understood that the composite wire, the coil comprising at least two composite wires, the cable comprising at least three composite wires, the medical device comprising such composite wire, coil and/or cable and the manufacturing method for a composite wire according to the independent claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

Description of the Drawings

The invention is now further illustrated using Figures, which are not to be considered as limiting the scope of the invention.

Figure 1 shows schematically a lead according to the invention.
Figure 2 shows schematically an apparatus for measuring fatigue resistance.
Figure 3 shows schematically a pacemaker comprising a lead according to the invention.
Figure 4 shows a cross sectional image of a wire of material according to example 2 (comparative).
Figure 5 shows a cross sectional image of a wire of material according to example 2 (comparative).
Figure 6 shows an analysis of elemental composition by energy dispersive x-ray spectroscopy of an inclusion in a wire of material according to example 2 (comparative).
Figure 7 shows a cross sectional image of a wire of material according to example 2 (comparative).
Figure 8 shows a cross sectional image of a wire of material according to example 2 (comparative).
Figure 9 shows a cross sectional image of a wire of material according to example 2a (comparative) with an Ag core.
Figure 10 shows a cross sectional image of a wire of material according to example 2a (comparative) with an Ag core.
Figure 11 shows an analysis of elemental composition by energy dispersive x-ray spectroscopy of an inclusion in a wire of material according to example 2a (comparative) with an Ag core.
Figure 12 shows a plot of fatigue results for a wire of material according to example 1 (inventive) and a wire of material according to example 2 (comparative).
Figure 13 shows a plot of fatigue results for a wire of material according to example 1a (inventive) with an Ag core and a wire of material according to example 2a (comparative) with an Ag core.
Figures 14 to 17 show cross sections of composite wires according to the invention.

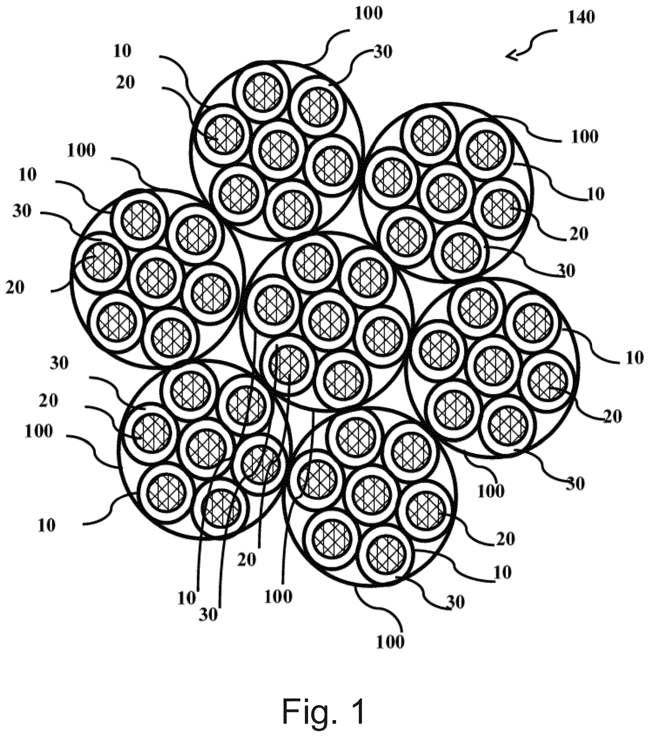

Figure 1 shows schematically a lead having a cable bundle 140, which comprises cables 100. In this example, the cables 100 each comprise 7 wires 10. Each wire comprises a first region 20 and a further region 30, wherein the first region 20 is interior to the region 30 along the length of the lead 140. The first region 20 is 41 area % of the cross sectional area the wire 10 and the further region is 59 area % of the cross sectional area of the wire 10, in each case based on the total cross sectional area of the wire 10. In this example, the first region 20 is silver. The further region 30 is a Cr, Ni, Mo and Co alloy as described above. In this example, the cable bundle 140 comprises 7 cables 100, each cable 100 comprising 7 wires 10. The invention is not limited to this arrangement. In particular, other arrangements of wires 10 in cables 100 and/or other arrangements of cable bundles 140 in leads are conceivable.

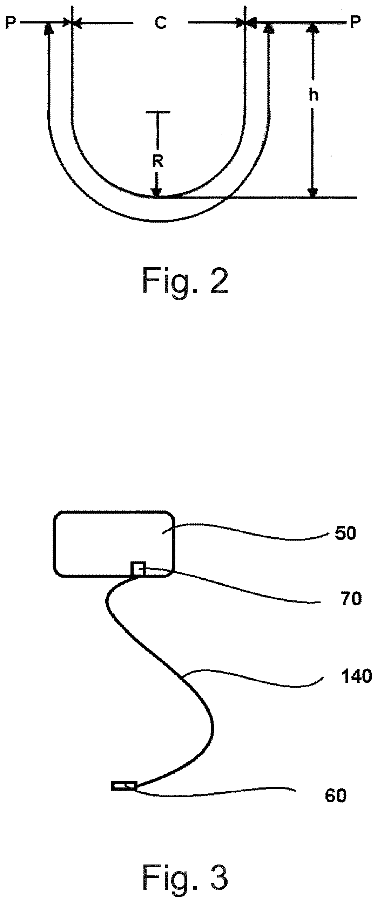

Figure 2 shows schematically an apparatus for measuring fatigue resistance.

Figure 3 shows schematically a pacemaker 50 with a pulse generator 70, and a lead 140 comprising an electrode 60. The lead 140 connects the pulse generator 70 and the heart tissue via the electrode 60.

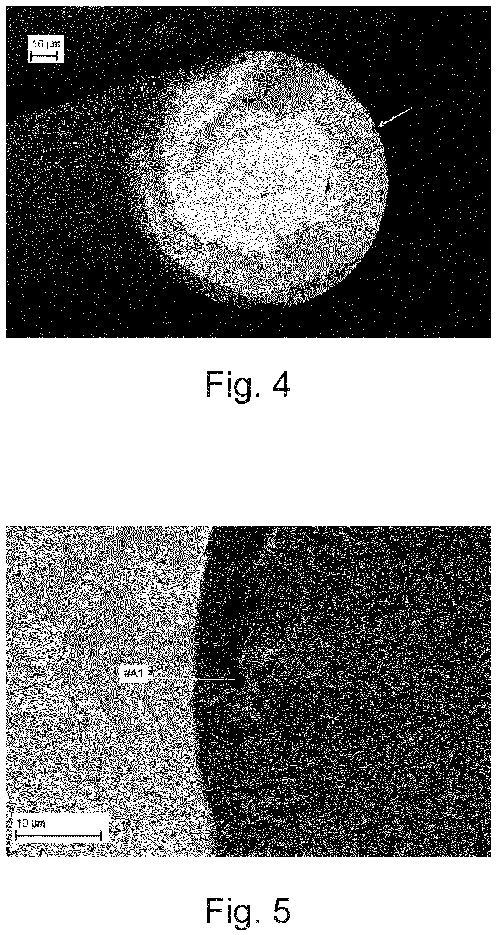

Figure 4 shows a cross sectional image of a wire of material according to example 2 (comparative) as observed by backscattered electron imaging according to the test method. A dark inclusion is indicated with an arrow.

Figure 5 shows a cross sectional image of a wire of material according to example 2 (comparative) as observed by backscattered electron imaging according to the test method. Figure 5 shows the same image as figure 4, but at higher magnification. A dark inclusion is indicated with the reference mark #A1.

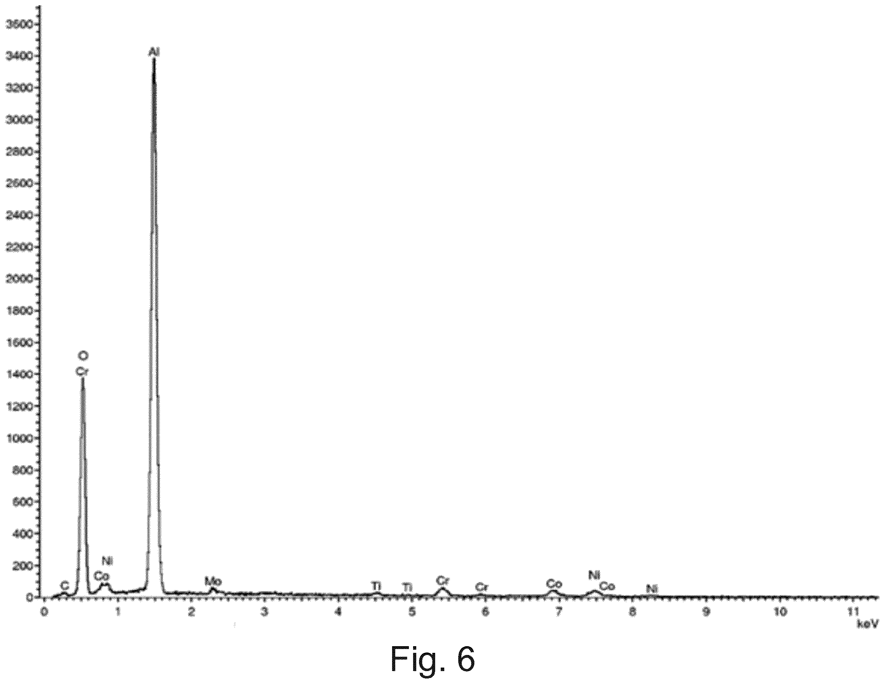

Figure 6 shows an analysis of elemental composition by energy dispersive x-ray spectroscopy according to the fracture surface analysis test method of the surface of an inclusion in a wire of material according to example 2 (comparative). The surface analysed is the inclusion indicated as #A1 in figure 5. In particular, the analysis shows the presence of Al and Mg impurities and also of entities with a Cr-O bond.

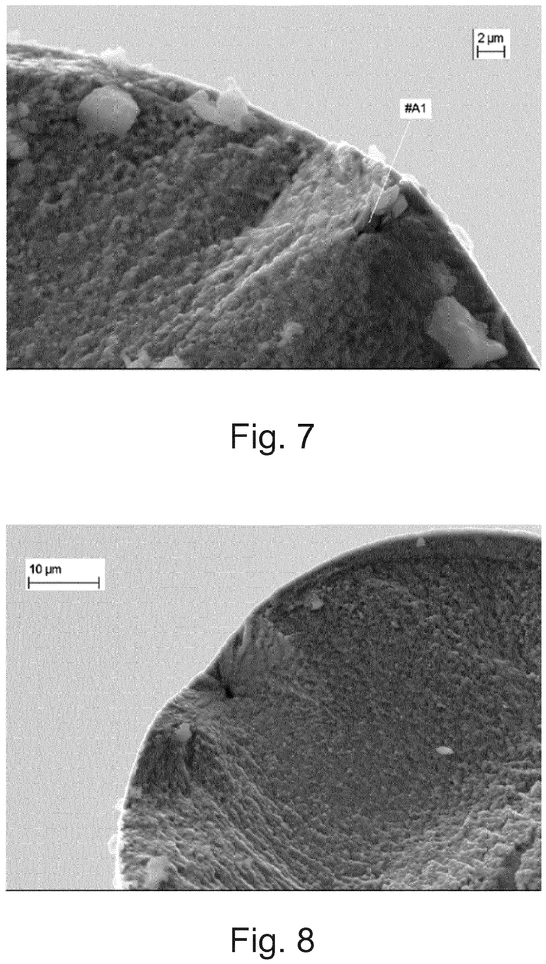

Figure 7 shows a cross sectional image of a wire of material according to example 2 (comparative) as observed by backscattered electron imaging according to the test method. A dark inclusion is indicated with the reference mark #A1.

Figure 8 shows a cross sectional image of a wire of material according to example 2 (comparative) as observed by backscattered electron imaging according to the test method. The surface shown in figure 8 is taken from the same slice as that of figure 7.

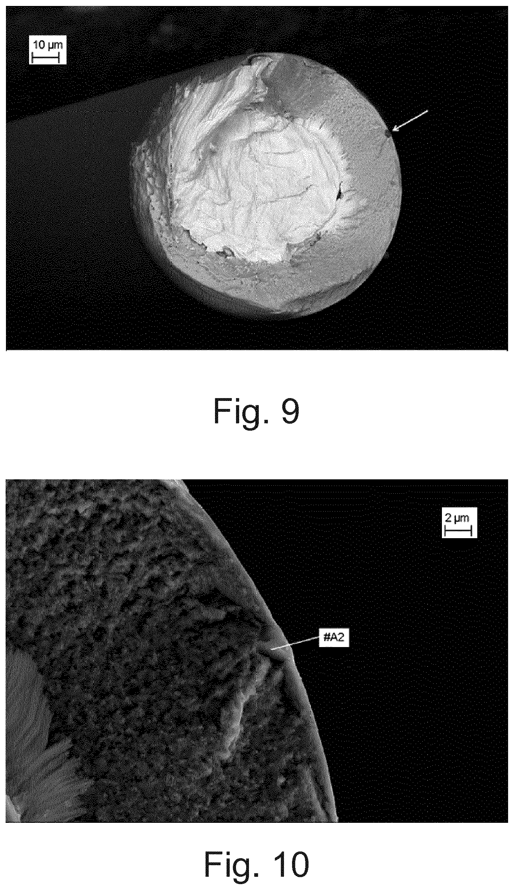

Figure 9 shows a cross sectional image of a wire of material according to example 2a (comparative) with an Ag core, as observed by backscattered electron imaging according to the test method. A dark inclusion is indicated with an arrow.

Figure 10 shows a cross sectional image of a wire of material according to example 2a (comparative) with an Ag core, as observed by backscattered electron imaging according to the test method. Figure 10 shows the same image as figure 9, but at higher magnification. A dark inclusion is indicated with the reference mark #A2.

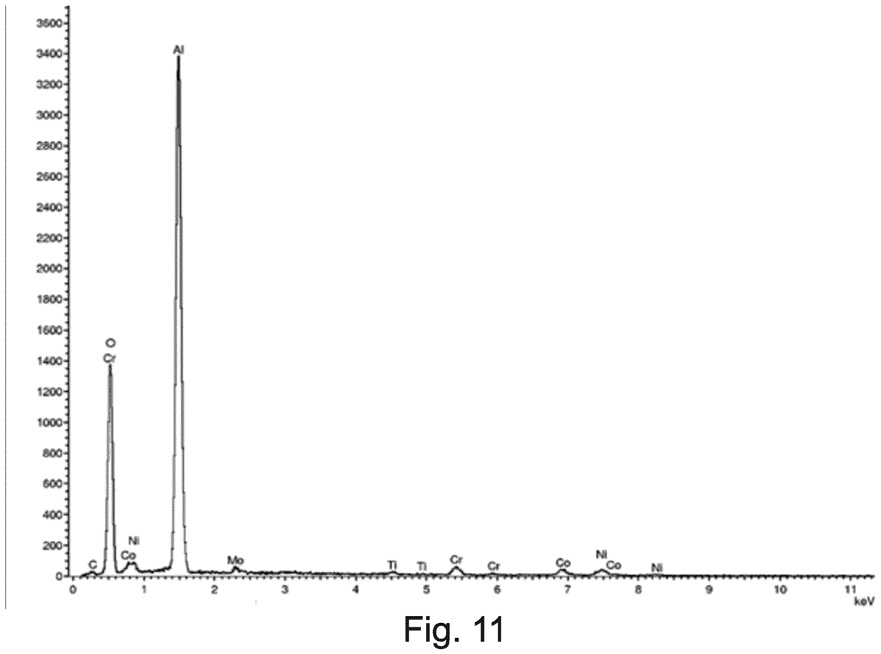

Figure 11 shows an analysis of elemental composition by energy dispersive x-ray spectroscopy according to the fracture surface analysis test method of the surface of an inclusion in a wire of material according to example 2a (comparative) with an Ag core. The surface analysed is the inclusion indicated as #A2 in figure 10. In particular, the analysis shows the presence of Al impurities and also of entities with a Cr-O bond.

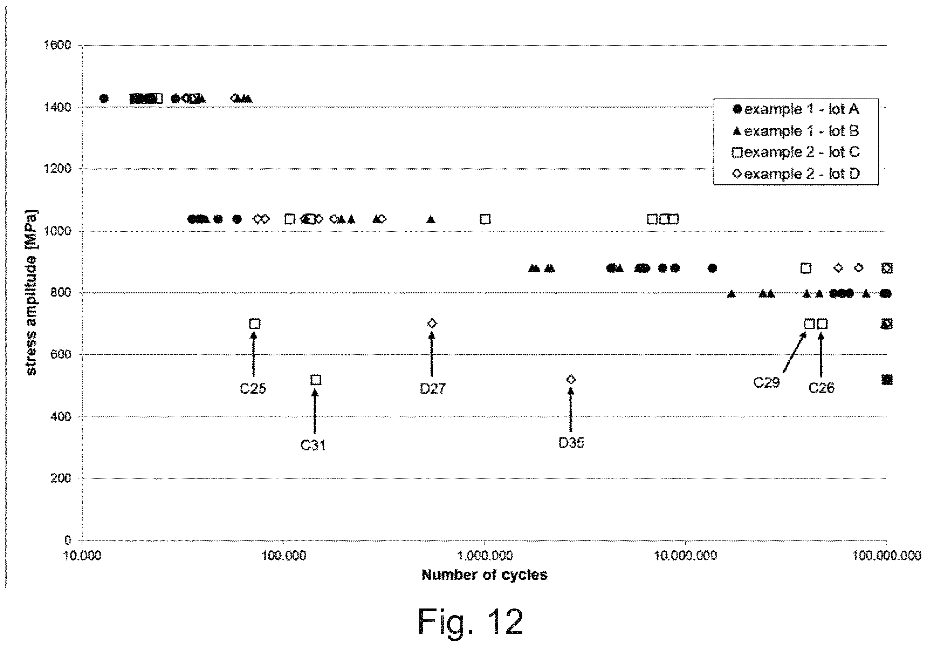

Figure 12 shows a plot of fatigue results for a wire of material according to example 1 (inventive) and a wire of material according to example 2 (comparative). For example 1 (inventive), results are shown for 2 lots, lot A as represented by a solid circle and lot B as represented by a solid triangle. For example 2 (comparative), results are shown for 2 lots, lot C as represented by a hollow square and lot D as represented by a hollow diamond. The number of cycles before failure is shown as dependent on the stress amplitude applied in the test. Outliers, which performed poorly are indicated with arrows.

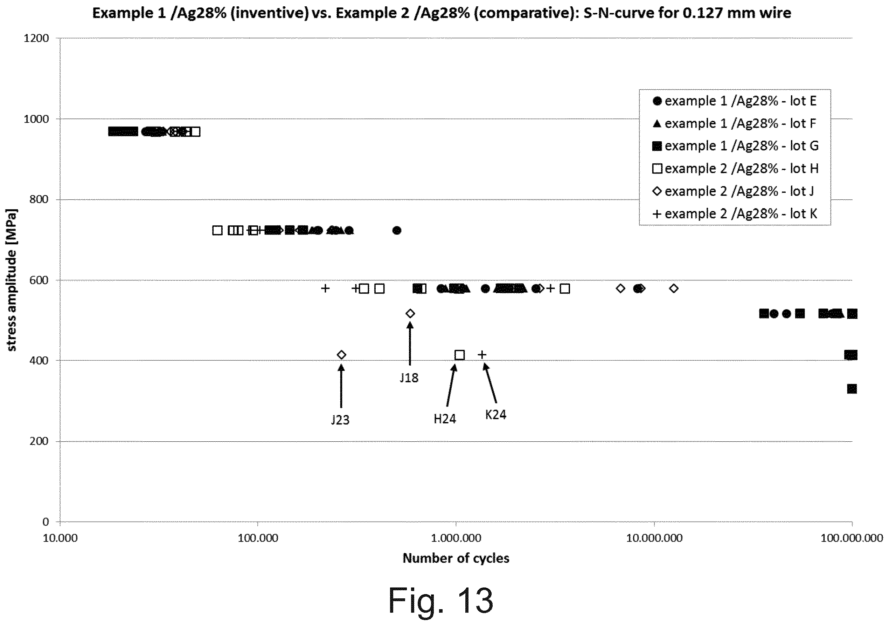

Figure 13 shows a plot of fatigue results for a wire of material according to example 1a (inventive) with an Ag core and a wire of material according to example 2a (comparative) with an Ag core. For example 1a (inventive), results are shown for 3 lots, lot E as represented by a solid circle, lot F as represented by a solid triangle and lot G as represented by a solid square. For example 2a (comparative), results are shown for 3 lots, lot H as represented by a hollow square, lot J as represented by a hollow diamond and lot K as represented by a cross. The number of cycles before failure is shown as dependent on the stress amplitude applied in the test. Outliers, which performed poorly are indicated with arrows.

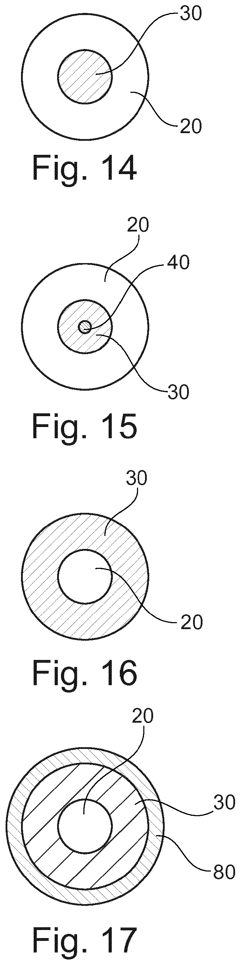

Figures 14 to 17 show cross sections of composite wires 10 according to the invention. They all comprise at least two different materials in form of a first part 20 and a second part 30.

In Figures 14 and 15, the first part 20 is a metal or an alloy to provide or enhance an electrical conductivity of the composite wire 10. The first part 20 surrounds the second part 30 and serves as a clad, cover, coating or the like. The first part 20 may also be biocompatible to improve the biocompatibility of the composite wire 10. The first part 20 may be Platinum or a Platinum based alloy.

The second part 30 comprises above mentioned Cr, Ni, Mo and Co alloy. The Cr, Ni, Mo and Co alloy is cleaner with less impurities and less and smaller inclusions which leads to an improved resistance to physical fatigue.

The composite wire 10 according to the invention combines the first metallic part with the second part 30 of a high purity Cr, Ni, Mo and Co alloy. The first metallic part provides electrical conductivity and the Cr, Ni, Mo and Co alloy and in particular, its high purity, provides an excellent fatigue resistance. As a result, the composite wire 10 meets at the same time electrical conductivity and fatigue resistance requirements for use in highly sensitive implanted applications such as the human brain.

As shown in Figure 15, the composite wire 10 may further comprise a core part 40, which is surrounded by the second part 30 when seen in the cross section. The core part 40 is again a metallic component out of Silver, Platinum and the like to further improve the electrical properties and in particular the electrical conductivity of the composite wire 10.

As described above, the first part 20 forms an outer shell for the second part 30 comprising the Cr, Ni, Mo and Co alloy. As described below, the second part 30 comprising the Cr, Ni, Mo and Co alloy may also form an outer shell for the first part 20.

In Figures 16 and 17, the second part 30 surrounds the first part 20 when seen in the cross section. The first part 20 is here a filling material and comprises e.g. Platinum, Tantalum, Gold, Copper, Silver and alloys thereof to enhance biocompatibility of the composite wire 10, provide or increase radiopacity and/or electrical conductivity. The second part 30 comprises above mentioned Cr, Ni, Mo and Co alloy to improve a fatigue resistance.

As shown in Figure 17, the composite wire 10 may further comprise a circumference part 80, which surrounds the second part 30 when seen in a cross section. The circumference part 80 may be biocompatible and comprises e.g. Platinum, a Platinum based alloy, a Platinum-Iridium alloy, a Platinum-Tungsten alloy, Gold, a Gold alloy, Tantalum, Titanium, a Titanium-Molybdenum alloy, a Titanium Aluminium Vanadium alloy and the like.

All composite wires 10 according to the invention may further comprise a coating (not shown) as outermost part surrounding all other parts. The coating may be applied on the first part 20, the second part 30 or the circumference part 80, whichever part is the outermost part of the composite wire 10. The coating may provide or enhance electrical insulation, electrical conductivity, mechanical properties, lubricity, biocompatibility and/or biostability of the composite wire 10.

The composite wires 10 as described above can be formed into coils or cables for use in medical lead applications such as pacemaker leads and neurostimulation leads.

Test Methods

Alloy composition

For a quantitative chemical analysis of the alloy, the following methods are used:

a) the main components of the alloy (Co, Cr, Ni, Mo) are measured by X-ray fluorescence XRF using the XRF Lab Report - S8 TIGER from the company BRUKER (Bruker AXS GmbH Ostliche Rheinbrückenstr. 49, 76187 Karlsruhe, Germany)

b) Trace elements present in the alloy (Mn, P, Si, Fe, Ti, Al, B, Mg, Ca, Ce, Ti) are measured by glow discharge mass spectrometry (GDMS) using the ASTRUM from Nu Instruments (Nu Instruments Limited, Unit 74, Clywedog Road South, Wrexham, LL13 9XS UK.)

c) Gas or non-metallic components in the alloy (H, O, C, N, S) are measured by carrier-gas hot extraction using the ONH836 from LECO (LECO Corporation, 3000 Lakeview Avenue, St. Joseph, Michigan 49085)

Leak rate

The leak rate of the furnace chamber is measured using the following procedure:

The Vacuum furnace chamber is evacuated to the required pressure by a vacuum pumping station. When the required pressure is reached, the pressure valve between the vacuum furnace chamber and the vacuum pumping station is closed. The pressure increase of the vacuum furnace chamber over a given length of time defines the leak rate of the equipment.

Fatigue resistance

Rotating beam fatigue testing was carried out using Valley Instruments model # 100 test machine (Figure 2) according to Valley Instruments Wire Fatigue Tester Model # 100 user manual (Valley Instruments (Division of Positool Technologies, Inc.), Brunswick, Ohio, USA. Fatigue Tester Model 100 Manual). The equipment consists of a synchronous motor rotating at 3600 rpm. For each test of a wire specimen, a sample having a predefined length is fixed in a custom fine-wire collet at one end, looped through a complete 180 degree turn and is placed at the other end in a low-friction bushing in which it is free to rotate. The synchronous motor of the test device is directly clocked by a counter where the number of cycles is shown in a LCD-display. The fatigue testers are equipped with a sensor to detect the wire fracture which automatically stops the timer, means the display of the timer shows the number of cycles until failure. If no fracture occurs within 100 Million cycles, the test is stopped.

Valley Instruments Wire Fatigue Tester Model # 100 user manual (Valley Instruments (Division of Positool Technologies, Inc.), Brunswick, Ohio, USA. Fatigue Tester Model 100 Manual) describes that a loop, formed by an elastic length held so that the axes of the specimen at the point of retention are exactly parallel, assumes a shape in which:

(1) The length of the loop is 2.19 times the base,
(2) The height of the arch is always 0.835 times the base,
(3) The minimum radius of the curvature occurs at the apex of the arch and is exactly 0.417 times the base, and
(4) The bending stress at the point of minimum curvature bears a simple reciprocal linear relationship to any of the four physical dimensions (length, height, base, and minimum curvature).

The following formulas express the exact relationship:

$$C = 1.198*E*d/S$$

$$h = 0.835*C$$

$$L = 2.19*C$$

$$R = 0.417*C$$

$$P = 0.141*E*d^4/C^2$$

Nomenclature:

C   = chuck to bushing distance

d　　= diameter of wire

h　　= height of loop

E　　= modulus of elasticity

L　　= length of wire external to chucks

R　　= minimum of radius of curvature

S　　= bending stress

P　　= bushing load or lateral force at the chuck

With the above listed formula, the bending stress S (at the peak of the loop) can be calculated by the following equation:

$$S = 1.198 * E * d / C$$

The machine set-up involves calculating the desired sample length and center distance using the modulus of elasticity of the material and equations developed by Valley Instruments Company (user manual).

Microscopic Inspection Method for Micro-cleanliness

Definition: Inclusions are defined as internal flaws or contaminations (such as nitrides or oxides) within the billet or rod from which the wire or tube is produced. The transverse inclusion size is defined as the largest dimension of an internal flaw measured on transverse cross-sections of the billet, rod or wire. The longitudinal inclusion size is defined as the largest dimension of an internal flaw measured on longitudinal cross-sections of the billet, rod or wire. A cross-section diametral line is defined as any line within the cross-section having a length equal to or greater than 95% of the true cross-section diameter.

General Test procedure:

a) Sectioning

For each material lot, the billet, rod or wire is to be sectioned at each end so that there are an equal number of cross sections sampled at the one end as there are samples at the other end (number of samples taken from each end shall differ by no more than one). The total number of cross sections samples depends on the diameter of the billet, rod or wire and is specified in Table 1. The length of each cross section is to be less than its diameter.

b) Imaging

For each billet, rod or solid wire cross-section, non-overlapping images are to be taken at 500X magnification along diametral lines so that the total examined area per sample is at least 1.77 mm$^2$. A cross-section diametral line is defined as any line within the cross-section having a length equal to or greater than 95% of the true cross-section diameter. Angular separation between two diametral lines on a cross- section shall be a minimum of 60 degrees. The number of images and the number of diametral lines depends on the diameter of the billet, rod or wire and is specified in Table 1.

The total number of images is shown in Table 1 and was calculated based on the number of images per sample and the number of samples.

c) Measurement

Each of the images is to be inspected to detect the presence of inclusions or strings of inclusions that exceed a size of 3.0 μm in their largest dimension. The image inspection may be accomplished either by manual examination or by automated scanning.

**Table 1**

| cross section diameter of billet, rod or wire | | Number of diametral lines per section (no requirement for tube samples) | Number of images per section | Number of cross-sections | | Total images per lot | |
|---|---|---|---|---|---|---|---|
| equal to or greater than [mm] | but no greater than [mm] | | | transverse | longitudinal | transverse | longitudinal |
| 2.54 | 3.80 | 5 | 40 | 12 | 12 | 480 | 480 |
| 3.81 | 5.71 | 3 | 40 | 12 | 12 | 480 | 480 |
| 5.72 | 11.42 | 2 | 40 | 12 | 12 | 480 | 480 |
| 11.43 | 13.96 | 1 | 40 | 12 | 12 | 480 | 480 |
| 13.97 | 17.14 | 1 | 48 | 10 | 10 | 480 | 480 |
| 17.15 | 21.58 | 1 | 60 | 8 | 8 | 480 | 480 |
| 21.59 | 27.93 | 1 | 80 | 6 | 6 | 480 | 480 |
| 27.94 | 33.01 | 1 | 96 | 5 | 5 | 480 | 480 |
| 33.02 | 43.17 | 1 | 120 | 4 | 4 | 480 | 480 |
| 43.18 | 57.14 | 1 | 160 | 3 | 3 | 480 | 480 |

Fracture surface analysis of wire samples

The test method to analyse fracture surfaces of fatigue tested samples was Scanning electron microscopy (SEM). A Zeiss Ultra 55 Gemini was used for the sample analysis of the present invention and comparative samples.
Two imaging modes were used to analyse and illustrate the tested samples.

a) SE: the detection of secondary electrons (SE) results in images with a well-defined, three-dimensional appearance. The surface topography can be illustrated in high resolution. Figures 5, 7, 8 and 10 are secondary electron images.
b) BSE: backscatter electrons (BSE) are used to detect contrast between areas with different chemical compositions. Heavy elements (high atomic number) backscatter electrons more strongly than light elements (low atomic number), and thus appear brighter in the image. Figures 4 and 9 are BSE images.

Energy-dispersive X-ray spectroscopy (EDS, EDX) was used for the elemental analysis of features (inclusions/particles) found on the fatigue resistance test samples. A high-energy beam of electrons is focused onto the location of the sample being analysed. This leads to the emission of characteristic X-rays which allows the elemental composition of the feature (inclusions/particles) to be measured. Figures 6 and 11 show EDX scans.

Examples

The MP35N heats were VIM-VAR melted, to minimize the impurity content and to obtain a sound ingot with good chemical uniformity and metallurgical properties. The chemistry of representative heats: Heat 1, Heat 2 and Heat 3 are listed in Table 3. The table also provides the chemistry of a VIM-VAR melted, commercially available MP35N alloy and for reference the chemical requirements per ASTM F562-13, a standard specification for wrought MP35N alloy. The major constituents of MP35N alloy are Co, Ni, Cr and Mo. The new alloy heats were melted in 2 steps. The first melting step was Vacuum Induction Melting (VIM). The VIM furnace consists of a water cooled vacuum melt chamber, an oxide ceramic crucible held in a cylindrical induction heating coil inside the melt chamber, an AC electric power supply, a vacuum pumping system, a raw material adding chamber and a cylindrical metal mold held below and offset from the crucible-induction coil assembly. The vacuum melt chamber, raw material adding chamber and vacuum pumping system are separated by isolation valves. The induction heating coil is water cooled. Electric current from the power supply passes through the induction heating coil creating a magnetic field inside the furnace. The magnetic field induces eddy currents inside the raw materials causing Joule heating. Joule heating raises the temperature of the raw materials to above their melting point. The magnetic field mixes the liquid raw materials to make a homogeneous alloy. The crucible is tilted to pour the liquid alloy from the crucible into the mold. The alloy cools to a solid in the mold under vacuum and is removed from the furnace. The alloy ingot is removed from the mold and it is prepared for re-melting.

For the example heats, 136 kilograms of elemental raw materials were placed in the furnace in proportions calculated to make the aim chemistry. The VIM furnace was closed and pumped down to ≤ 0.00001 bar. A leak-up rate was measured after reaching the desired vacuum pressure level to ensure a vacuum tight furnace. The leak-up rate was ≤ 0.00001 bar/min. Electric power was applied to the induction heating coil. Once the melt was in progress, the vacuum level was recorded at specified intervals to monitor the progress of melting and the mixing and reaction of all of the raw materials. When the reactions ceased as indicated by a constant vacuum pressure level, the heat was poured into a 152.4 mm diameter cylindrical mold.

Each heat was subsequently re-melted by a Vacuum Arc Re-melting (VAR) process to make an 203.2 mm diameter ingot. The VAR furnace consists of water cooled vacuum chamber, a 203.2 mm diameter water cooled copper crucible, a direct current electric power supply, a vacuum pumping system, isolation valves and a computer based electrical system to monitor and control the application of current to the electrode inside the vacuum chamber. The furnace was pumped down to ≤ 0.000006 bar before carrying out the leak-up rate test. A leak rate of ≤ 0.000006 bar /min was obtained. The electrode was moved to a close proximity to the bottom of the crucible. Electric power was applied at a level to cause an electric arc to be struck between the crucible bottom and the alloy electrode. The electric arc causes the electrode to melt and drip into the bottom of the crucible creating a liquid metal pool that solidifies as the arc moves away from the molten pool. The process was continued at a controlled rate until the electrode was consumed. The power was turned off and the ingot was cooled under vacuum. The ingot was removed from the furnace for processing to product.

The as-cast ingot was charged into a gas-fired front opening box furnace with ambient air atmosphere. The furnace was preset to a temperature of 815°C. Upon equilibration of furnace temperature, the ingot was held for additional 4 hours prior to raising the furnace temperature. The ingot was then heated to 1177°C at a heating rate of 200 K per hour. The ingot was held for 7 hours at 1177°C for homogenization. After homogenization, the ingot was hot rolled from 203 mm to 137 mm round cornered square (RCS) billet using a 559 mm diameter Morgenshammer Mill operating at ambient temperature. The Morgenshammer Mill is a manually operated tilt table mill with 3 high rolls allowing heavy bar to be rolled alternately between the bottom and middle roll and the top and middle roll. After hot rolling the RCS billet was air cooled, abrasively ground by hand to remove surface imperfections and cut to square the ends. The billet was reheated and hot rolled to 51 mm RCS at 1177°C on the 559 mm Morgenshammer Mill. The RCS was cut to shorter lengths of final rolling on a hand operated 406 mm diameter Morgenshammer Mill with 3 high rolls. All bar manipulation on this mill is done by hand at floor level. The RCS was reheated at 1177°C and rolled to 33.4 mm round bars and air cooled to ambient temperature. The rolled bars were then reheated to 1038°C and held for 30 minutes for hot rotary straightening. After straightening, the bars were air cooled to room temperature. The bars were rough centerless ground, ultrasonic tested for voids and then centerless ground to final size.

For manufacturing of clad-wires, the grinded bars were gun-drilled to produce hollows for subsequent tube drawing. Tubes were filled with Ag-rods and cold-drawn using diamond dies and mineral oil. For a final wire diameter of 127 μm, the last intermediate annealing was carried out at a wire diameter of 157.5 μm at 900 - 950 °C in Argon atmosphere. From the last intermediate annealing until the final diameter of the wire, 35% cold-work were applied. Three wire lots were manufactured having UTS values of 1456, 1469 and 1474 MPa. For bare wire, the bars were further hot-rolled to 0.2 inch outer diameter followed by cold-drawing. For 102 μm final size wire, the last intermediate annealing was carried out at a wire diameter of 122 μm at 1100 °C in Argon atmosphere to apply 30% cold-work to the final size. Two wire lots were manufactured having UTS values of 1870 and 1875 MPa. The wires of inventive example 1 (Lots A & B) and the cladded wires of inventive example 1a (Lots E, F & G) were made using the alloy of Heat 1 in table 3. The wires of comparative example 2 (lots C & D) and the cladded wires of comparative example 2a (lots H, J & K) were made from the alloy of the commercial heat in table 3 obtained from Fort Wayne Metals, Inc., USA under the trade name 35 NLT®.

**Table 2**

| Material | Example 1 (inventive) | | Example 1a with 28 % Ag (inventive) | | |
|---|---|---|---|---|---|
| Batch | Lot A | Lot B | Lot E | Lot F | Lot G |
| UTS [MPa] | 1870 | 1875 | 1456 | 1469 | 1474 |
| YM [GPa] | 190 | 191 | 121 | 121 | 122 |
| Elongation [%] | 2.8 | 2.9 | 2.2 | 2.3 | 2.3 |

The processed alloy was also obtainable from SAES Smart Materials, Inc. Alloys for the further examples were acquired from SAES Smart Materials, Inc.

**Table 3**

| Element | Heat 1 | Heat 2 | Heat 3 | Commercial Heat | ASTM F-562-13 |
|---|---|---|---|---|---|
| | Wt. % | Wt. % | Wt. % | Wt. % | Wt. % |
| c | 0.0039 | 0.0091 | 0.0106 | 0.005 | < 0.0250 |
| B | 0.000065 | 0.000067 | 0.000008 | 0.01 | < 0.015 |
| P | 0.00018 | 0.000095 | 0.000056 | 0.001 | < 0.015 |
| S | 0.00056 | 0.00026 | 0.00036 | 0.001 | < 0.010 |
| Mn | 0.00028 | 0.00021 | 0.00013 | 0.017 | < 0.15 |
| Si | 0.0042 | 0.0053 | 0.0061 | 0.034 | < 0.15 |
| Al | 0.00023 | 0.00054 | 0.00043 | 0.023 | NA |
| Mg | <0.000001 | 0.000003 | 0.000005 | 0.001 | NA |
| Ca | <0.000005 | <0.000005 | <0.000005 | NA | NA |
| Ce | <0.000001 | <0.000001 | <0.000001 | NA | NA |
| Fe | 0.021 | 0.023 | 0.023 | 0.08 | < 1 |
| Ti | 0.00017 | 0.000038 | 0.000023 | 0.001 | < 1 |
| O | 0.0085 | 0.0056 | 0.0035 | 0.0021 | NA |
| N | 0.0022 | 0.0009 | 0.0007 | 0.0022 | NA |
| Cr | 19.6 | 19.7 | 20 | 20.62 | 19 - 21 |
| Ni | 35.7 | 34.8 | 34.9 | 34.91 | 33 - 37 |
| Mo | 10 | 9.93 | 9.7 | 9.47 | 9 - 10.5 |
| Co | balance | balance | balance | balance | balance |
| Microscopic Inspection for Microcleanliness of the alloy | | | | | |

The microscopic inspection for microcleanliness of the inventive alloy (example 1 and example 1a with an Ag core) and of the comparative alloy (example 2 and example 2a with an Ag core) was carried out according to the procedure and test method described above. Of 4 rods with an outer diameter of 31.75 mm, 5 transverse and 5 longitudinal sections were taken according to table 1 and metallographically prepared. The sections included a continuous plane from two surface locations and through the approximated center of the bar. The metallographically prepared sections were examined in the as-polished condition by scanning electron microscopy (SEM) using backscattered electron imaging (BEI). In BEI, the brightness of sample features is proportional to the atomic weight of the elements constituting those features. Thus, in BEI, present inclusions consisting of heavier elements than the surrounding matrix material appear brighter than the matrix material. Inclusions consisting of lighter elements than the surrounding matrix material appear darker than the matrix material. Since nonmetallic inclusions (e.g. oxide or nitride inclusions) consist of lighter elements than the alloys of example 1 and example 2, in BEI these ceramic inclusions appear darker than the surrounding matrix material. Images were acquired at a magnification of 500X along a diametral line extending across the entire bar. Analysis of features darker and brighter than the background was conducted on the images using image analysis software to determine the maximum dimension for each detected feature. The largest dimension and area were recorded for each individual feature. The inclusions were categorized by largest dimension into 1 $\mu$m groups up to 14 $\mu$m. The total area of the dark and bright features was also calculated. Inclusions greater than 14 $\mu$m were also counted. Features smaller than 3.0 $\mu$m were not included in the measurements.

For each section, forty-eight fields of view were evaluated. For each direction, longitudinal and transverse, 480 images with a total area of 22.6 mm$^2$ were evaluated. The samples contained features that appeared darker and brighter than the bulk material using backscattered electron imaging. The darker features have a lower mean atomic number than the background and the brighter features have a higher mean atomic number than the background.

Results of the inclusion analysis of example 1 are shown in tables 4-6. Results of the inclusion analysis of example 2 are shown in tables 7-10. Image fields showing typical dark (ceramic) inclusions are shown in Figures 4,5, 7-10.

Alloy of the present invention (example 1):

### TABLE 4 - FEATURE COUNT TOTALS / EXAMPLE 1

| | Number of Features | | | |
|---|---|---|---|---|
| Largest Dimension [$\mu$m] | Longitudinal | | Transverse | |
| | Dark | Bright | Dark | Bright |
| 3.0       - 3.9 | 15 | 0 | 14 | 0 |
| 4.0       - 4.9 | 4 | 0 | 2 | 0 |
| 5.0       - 5.9 | 2 | 0 | 1 | 0 |
| 6.0       - 6.9 | 0 | 0 | 0 | 0 |
| 7.0       - 7.9 | 0 | 0 | 0 | 0 |
| 8.0       - 8.9 | 0 | 0 | 0 | 0 |
| 9.0       - 9.9 | 0 | 0 | 0 | 0 |
| 10.0       - 10.9 | 0 | 0 | 0 | 0 |
| 11.0       - 11.9 | 0 | 0 | 0 | 0 |
| 12.0       - 12.9 | 0 | 0 | 0 | 0 |
| 13.0       - 13.9 | 0 | 0 | 0 | 0 |
| 14.0       - 14.9 | 0 | 0 | 0 | 0 |
| >14.9 | 0 | 0 | 0 | 0 |
| | | | | |
| Total | 21 | 0 | 17 | 0 |

### TABLE 5 - TOTAL INCLUSION AREA MEASUREMENTS FOR EXAMPLE 1

| | Area of Inclusions > 3 $\mu$m in Length for Examination Region | | | | | |
|---|---|---|---|---|---|---|
| | Darker | | Brighter | | All | |
| Sample | Total | Percent of Total Area | Total | Percent of Total Area | Total | Percent of Total Area |
| | [$\mu$m$^2$] | [%] | [$\mu$m$^2$] | [%] | [$\mu$m$^2$] | [%] |
| Longitudinal | 121 | 0.0006 | 0 | 0.0000 | 121 | 0.0006 |
| Transverse | 97 | 0.0005 | 0 | 0.0000 | 97 | 0.0005 |

### TABLE 6 - LONGEST DARK FEATURES FOR EXAMPLE 1

| | Feature Dimensions, [$\mu$m] | | |
|---|---|---|---|
| Number | Length | Breadth | Direction |
| 1 | 5.6 | 3.0 | Longitudinal |
| 2 | 5.4 | 3.7 | Longitudinal |
| 3 | 5.4 | 2.9 | Longitudinal |
| 4 | 4.9 | 2.2 | Longitudinal |

(continued)

| | Feature Dimensions, [μm] | | |
|---|---|---|---|
| Number | Length | Breadth | Direction |
| 5 | 4.7 | 3.6 | Longitudinal |
| 6 | 4.6 | 1.9 | Longitudinal |
| 7 | 4.5 | 2.7 | Longitudinal |
| 8 | 4.5 | 2.4 | Longitudinal |
| 9 | 4.1 | 2.4 | Longitudinal |
| 10 | 3.9 | 3.0 | Longitudinal |

example 2 (comparative):

### TABLE 7 - FEATURE COUNT TOTALS / BARS 1-10 / ALL SAMPLES

| | Number of Features | | | |
|---|---|---|---|---|
| Largest Dimension [μm] | Longitudinal | | Transverse | |
| | Dark | Bright | Dark | Bright |
| 3.0 - 3.9 | 25 | 21 | 6 | 46 |
| 4.0 - 4.9 | 19 | 7 | 3 | 11 |
| 5.0 - 5.9 | 7 | 1 | 1 | 1 |
| 6.0 - 6.9 | 6 | - | - | 1 |
| 7.0 - 7.9 | 7 | - | - | - |
| 8.0 - 8.9 | 4 | - | - | - |
| 9.0 - 9.9 | 1 | - | - | - |
| 10.0 - 10.9 | 2 | - | - | - |
| 11.0 - 11.9 | 2 | - | - | - |
| 12.0 - 12.9 | - | - | - | - |
| 13.0 - 13.9 | - | - | - | - |
| 14.0 - 14.9 | - | - | - | - |
| >14.9 | 4 | - | - | - |
| Total | 77 | 29 | 10 | 59 |

### TABLE 8 - TOTAL INCLUSION AREA MEASUREMENTS FOR EXAMPLE 2

| | Area of Inclusions > 3 μm in Length for Examination Region | | | | | |
|---|---|---|---|---|---|---|
| | Darker | | Brighter | | All | |
| Sample | Total | Percent of Total Area | Total | Percent of Total Area | Total | Percent of Total Area |
| | [μm²] | [%] | [μm²] | [%] | [μm²] | [%] |
| Longitudinal | 409 | 0.0018 | 75 | 0.0003 | 484 | 0.0021 |
| Transverse | 69 | 0.0003 | 152 | 0.0007 | 221 | 0.0010 |

**TABLE 9 - LONGEST DARK FEATURES FOR EXAMPLE 2**

|  | Feature Dimensions, [μm] |  |  |
| --- | --- | --- | --- |
| Number | Length | Breadth | Direction |
| 1 | 33.4 | 1.9 | Longitudinal |
| 2 | 18.9 | 1.6 | Longitudinal |
| 3 | 17.8 | 2.3 | Longitudinal |
| 4 | 15.4 | 1.4 | Longitudinal |
| 5 | 11.8 | 1.0 | Longitudinal |
| 6 | 11.1 | 1.1 | Longitudinal |
| 7 | 10.6 | 1.0 | Longitudinal |
| 8 | 10.3 | 1.8 | Longitudinal |
| 9 | 9.5 | 1.5 | Longitudinal |
| 10 | 8.9 | 2.2 | Longitudinal |

**TABLE 10 - LONGEST BRIGHT FEATURES FOR EXAMPLE 2**

|  | Feature Dimensions, [μm] |  |  |
| --- | --- | --- | --- |
| Number | Length | Breadth | Direction |
| 1 | 6.0 | 1.6 | Transverse |
| 2 | 5.6 | 2.8 | Longitudinal |
| 3 | 5.1 | 1.8 | Transverse |
| 4 | 4.9 | 2.3 | Transverse |
| 5 | 1.9 | 1.8 | Transverse |
| 6 | 1.0 | 1.8 | Longitudinal |
| 7 | 4.6 | 1.3 | Transverse |
| 8 | 4.5 | 1.2 | Transverse |
| 9 | 4.4 | 1.6 | Longitudinal |
| 10 | 4.4 | 0.9 | Transverse |

According to Table 8 of example 2 (comparative), the total area of dark inclusions found is 478 $\mu m^2$ (409 $\mu m^2$ in longitudinal direction and 69 $\mu m^2$ in transverse direction). According to Table 5 of example 1 (inventive), the total area of dark inclusions found is only 218 $\mu m^2$ (121 $\mu m^2$ in longitudinal direction and 97 $\mu m^2$ in transverse direction). So the amount of dark inclusions (Percent of total area) in example 1 (inventive) is only 4.8 ppm (0.00048 %) while in example 2 (comparative) the amount of dark inclusions is 11 ppm (0.0011 %). In terms of inclusions (micro-cleanliness) this means that example 1 (inventive) is more than 2 times cleaner than example 2 (comparative).

Fatigue Test Results

Two lots of wire of example 1 (dia. 102 $\mu m$) were tested against two lots of wire or example 2 (same diameter - 102 $\mu m$) having comparable mechanical properties (UTS of 1862 - 1875 MPa).

**Table 11**

| Material | Example 1 (inventive) | | Example 2 (comparative) | |
|---|---|---|---|---|
| Batch | Lot A | Lot B | Lot C (Fig. 5 & 6) | Lot D |
| UTS [MPa] | 1870 | 1875 | 1862 | 1871 |
| YM [GPa] | 190 | 191 | 190 | 190 |
| Elongation [%] | 2.8 | 2.9 | 2.7 | 2.8 |

At an applied stress of 700 MPa, the wire of all four lots reached the fatigue endurance limit, means the wire does not fail and tests are stopped after 100 Million cycles. While the wire of example 1 showed no outliers at 700 MPa and below, 4 samples of example 2 failed at less than 2.7 Million cycles and two other samples ran 40 - 50 Million cycles. All other samples tested at an applied stress of 700 MPa and below survived 100 Million cycles without rupture. For Example 2 wire lot C, sample C25 tested at an applied stress of 700 MPa broke after only 71,790 cycles and sample C31 tested at an applied stress of 520 MPa broke after only 145,260 cycles. Sample C26 tested at an applied stress of 700 MPa broke after 47,547,540 cycles and sample C29 tested at an applied stress of 700 MPa broke after 41,282,990 cycles. For example 2 wire lot D, sample D27 tested at an applied stress of 700 MPa broke after only 549,227 cycles and sample D35 tested at an applied stress of 520 MPa broke after only 2,689,952 cycles. SEM-images of sample C25 shows an inclusion at the fracture surface. In EDX analysis, high peaks for Aluminium, Magnesium, Chromium and Oxygen were found. This mixed-oxide inclusion was identified as the crack initiation point for the early failure of this sample. An SEM-image of sample D35 also shows an inclusion at the fracture surface. Again, in EDX analysis, high peaks for Aluminium, Magnesium, Chromium and Oxygen were found. Also this mixed-oxide inclusion can be identified as the crack initiation point for the early failure of this sample. SEM investigations of samples C31 and D27 also showed oxide-inclusions at the fracture surface which were identified causing the early failure. For both samples, the same elements (Aluminium, Magnesium, Chromium, Oxygen) show high peaks in EDX analysis for these two samples.

**Table 12**

| stress level [MPa] | Example 1 (inventive) (Lot A) | Example 1 (inventive) (Lot B) | Example 2 (comparative) (Lot C) | Example 2 (comparative) (Lot D) |
|---|---|---|---|---|
| | No. of cycles | No. of cycles | No. of cycles | No. of cycles |
| 1430 | 21092 | 66720 | 20700 | 35130 |
| 1430 | 12740 | 63781 | 22140 | 57120 |
| 1430 | 28919 | 59140 | 36120 | 32460 |
| 1430 | 19334 | 37942 | 23550 | 35010 |
| 1430 | 18119 | 39178 | 18270 | 28996 |
| 1430 | 21983 | 22380 | 18150 | 33313 |
| 1040 | 38670 | 128644 | 136680 | 149971 |
| 1040 | 58474 | 218106 | 1004289 | 308580 |
| 1040 | 46980 | 194108 | 8656363 | 80766 |
| 1040 | 34806 | 41310 | 107640 | 178770 |
| 1040 | 38045 | 539089 | 7856526 | 74323 |
| 1040 | 39120 | 290101 | 6852041 | 127650 |
| 880 | 5888420 | 4715690 | 39485962 | 72523366 |
| 880 | 4233055 | 5816670 | 102020453 | 100000000 |
| 880 | 6324748 | 2144125 | 100800000 | 6171230 |
| 880 | 13571905 | 2068519 | 114000000 | 4407264 |
| 880 | 8824316 | 1725656 | 101000000 | 102000000 |

(continued)

| stress level [MPa] | Example 1 (inventive) (Lot A) | Example 1 (inventive) (Lot B) | Example 2 (comparative) (Lot C) | Example 2 (comparative) (Lot D) |
|---|---|---|---|---|
| | No. of cycles | No. of cycles | No. of cycles | No. of cycles |
| 880 | 7680042 | 1815390 | 100000000 | 57462763 |
| 800 | 104700000 | 40051186 | - | - |
| 800 | 96874223 | 16938278 | - | - |
| 800 | 59716187 | 26518613 | - | - |
| 800 | 54411683 | 79084889 | - | - |
| 800 | 64971417 | 46467823 | - | - |
| 800 | 100000000 | 24231864 | - | - |
| 700 | 103000000 | 105000000 | 71790 | 100000000 |
| 700 | 109852488 | 97119288 | 47547540 | 100000000 |
| 700 | 101589761 | 107000000 | 100000000 | 549227 |
| 700 | 101623566 | 104000000 | 100000000 | 101337563 |
| 700 | 102000000 | 101000000 | 41282990 | 100000000 |
| 700 | 103000000 | 103000000 | 100000000 | 100000000 |
| 520 | 100000000 | 100000000 | 145260 | 100000000 |
| 520 | 118987000 | 106000000 | 110800000 | 110800000 |
| 520 | 102064330 | 102000000 | 101000000 | 101000000 |
| 520 | 100963860 | 100613526 | 100500000 | 100500000 |
| 520 | 100845911 | 100845911 | 108000000 | 2689952 |
| 520 | 101009712 | 101006089 | 100000000 | 112000000 |

These fatigue test results are plotted in Figure 12. As can be seen from this plot, lots C and D (comparative) show significantly more undesired outliers that for lots A and B (inventive).

Three lots of example 1a/Ag28% wire (diameter 127 μm) were also tested against three lots of example 2a wire (same diameter - 127 μm). All six wire lots have comparable mechanical properties (UTS of 1456 - 1475 MPa).

**Table 13**

| - | Example 1a + 28 wt. % Ag (inventive) | | | Example 2a + 28 wt. % Ag (comparative) | | |
|---|---|---|---|---|---|---|
| Batch | Lot E | Lot F | Lot G | Lot H | Lot J (Fig. 10 & 11) | Lot K |
| UTS [MPa] | 1456 | 1469 | 1474 | 1460 | 1462 | 1475 |
| YM [GPa] | 121 | 121 | 122 | 121 | 122 | 122 |
| Elongation [%] | 2.2 | 2.3 | 2.3 | 2.1 | 2.0 | 2.3 |

At an applied stress of 414 MPa, the wire of all four lots reached the fatigue endurance limit, means the wire does not fail and tests are stopped after 100 Million cycles. While example 1a /Ag28% wire showed no outliers at 414 MPa and below, 4 samples of example 2a /Ag28% wire failed at less than 1.4 Million cycles. All other samples tested at an applied stress of 414 MPa and below survived 100 Million cycles without rupture. For example 2a /Ag28% wire lot H, sample H24 tested at an applied stress of 414 MPa broke after only 1,041,679 cycles. Sample J18 tested at an applied stress of 518 MPa broke after 588,028 cycles and sample J23 tested at an applied stress of 414 MPa broke after 263,488 cycles. Sample K24 tested at an applied stress of 414 MPa broke after 1,355,189 cycles. As an example, SEM-images of sample J23 show an inclusion at the fracture surface. In EDX analysis, high peaks for Aluminium, Magnesium, Chromium and Oxygen were found. This mixed-oxide inclusion was identified as the crack initiation point for the early failure of this sample. SEM investigations of samples H24, J18 and K24 also showed oxide-inclusions at the fracture surface which were identified causing the early failure. For all three samples, the same elements (Aluminium, Magnesium, Chromium, Oxygen) show high peaks in EDX analysis for these three samples.

**Table 14**

| | Example 1a + 28 wt. % Ag | | | Example 2a + 28 wt. % Ag | | |
|---|---|---|---|---|---|---|
| | lot E | lot F | lot G | lot H | lot J | lot K |
| stress level [MPa] | No. of cycles | No. of cycles | No. of cycles | No. of cycles | No. of cycles | No. of cycles |
| 969 | 31,887 | 33,065 | 21,487 | 47,982 | 38,148 | 32,926 |
| 969 | 29,321 | 28,116 | 23,461 | 43,661 | 41,085 | 20,818 |
| 969 | 32,555 | 29,941 | 28,763 | 30,298 | 33,187 | 32,299 |
| 969 | 26,918 | 23,418 | 18,464 | 37,888 | 36,247 | 38,901 |
| 969 | 22,089 | 30,467 | 20,198 | 43,092 | 41,944 | 42,978 |
| 725 | 246,766 | 231,412 | 114,746 | 74,414 | 235,494 | 109,597 |
| 725 | 199,054 | 189,441 | 123,746 | 79,498 | 128,377 | 92,419 |
| 725 | 287,665 | 262,994 | 168,374 | 94,638 | 118,922 | 91,877 |
| 725 | 200,822 | 186,242 | 145,355 | 75,062 | 162,522 | 102,834 |
| 725 | 500,045 | 290,377 | 169,176 | 62,082 | 238,611 | 99,864 |
| 580 | 1,405,296 | 1,612,743 | 979,651 | 409,644 | 6,780,968 | 311,974 |
| 580 | 1,077,510 | 1,131,168 | 1,846,396 | 668,132 | 12,545,505 | 8,369,715 |
| 580 | 8,201,513 | 993,416 | 1,684,673 | 1,031,447 | 1,945,002 | 3,001,478 |
| stress level [MPa] | No. of cycles | No. of cycles | No. of cycles | No. of cycles | No. of cycles | stress level [MPa] |
| 580 | 2,511,763 | 2,197,173 | 639,464 | 342,282 | 2,639,912 | 219,634 |
| 580 | 841,436 | 884,196 | 2,076,465 | 3,539,353 | 8,566,249 | 2,009,899 |
| 518 | 40,051,186 | 86,414,732 | 71,763,385 | 100,000,000 | 100,000,000 | 100,000,000 |
| 518 | 100,000,000 | 78,411,674 | 83,944,821 | 100,000,000 | 100,000,000 | 100,000,000 |
| 518 | 79,084,889 | 100,000,000 | 35,946,337 | 100,000,000 | 588,028 | 100,000,000 |
| 518 | 46,467,823 | 100,000,000 | 100,000,000 | 100,000,000 | 100,000,000 | 100,000,000 |
| 518 | 100,000,000 | 87,867,423 | 54,676,179 | 100,000,000 | 100,000,000 | 100,000,000 |
| 414 | 100,000,000 | 100,000,000 | 100,000,000 | 100,000,000 | 100,000,000 | 100,000,000 |
| 414 | 100,000,000 | 100,000,000 | 100,000,000 | 100,000,000 | 100,000,000 | 100,000,000 |
| 414 | 100,000,000 | 100,000,000 | 98,674,345 | 100,000,000 | 263,488 | 100,000,000 |
| 414 | 100,000,000 | 100,000,000 | 100,000,000 | 1,041,679 | 100,000,000 | 1,355,189 |
| 414 | 100,000,000 | 100,000,000 | 96,674,523 | 100,000,000 | 100,000,000 | 100,000,000 |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |

These fatigue test results are plotted in Figure 13. As can be seen from this plot, lots H, J and K (comparative) show significantly more undesired outliers that for lots E, F and G (inventive).

Pacemaker lead

A wire with thickness 25 $\mu$m was prepared according to the method described above and with compositions of the alloy as given in table 3. The wires were arranged into a lead as described in figure 1. The leads were tested for fatigue resistance and for impurity inclusions. The results are shown in table 15.

**Table 15**

| Example | Fatigue resistance | Purity from inclusions |
|---|---|---|
| Heat 1 | ++ | ++ |
| Heat 2 | ++ | ++ |
| Heat 3 | ++ | ++ |
| Commercial heat | - | - |
| ++ = very good, - = poor | | |

Reference list

| | |
|---|---|
| 10 | Composite wire |
| 20 | First part or region |
| 30 | Further, second part or region |
| 40 | Core part |
| 50 | Medical device or pacemaker |
| 60 | Electrode unit |
| 70 | Electronic unit |
| 80 | Circumference part |
| 100 | Cable |
| 110 | First mass |
| 120 | Body |
| 130 | Precursor |
| 140 | Lead/Cable bundle |
| C | chuck to bushing distance |
| H | height of loop |
| R | minimum of radius of curvature |
| P | bushing load or lateral force at the chuck |
| #A1 | Dark inclusion |
| #A2 | Dark inclusion |

**Claims**

**1.** A composite wire (10), comprising

- a first part (20), and
- a second part (30), forming an outer shell for the first part (20),

wherein the first part (20) is a metallic component, and

wherein the second part (30) comprises an alloy consisting of the following alloy components:

a) Cr in the range from 10 to 30 wt. %;
b) Ni in the range from 20 to 50 wt. %;
c) Mo in the range from 2 to 20 wt. %; and
d) Co in the range from 10 to 50 wt. %;

wherein the Al content of the alloy is less than 0.01 wt. %;
wherein each wt. % is based on the total weight of the alloy,
wherein the alloy further contains
less than 0.005 wt. % Mg,
less than 0.005 wt. % Ca,
less than 0.005 wt. % Ce,
less than 0.1 wt. % Ti, from 0.0001 to 1 wt. % Fe,
less than 0.1 wt. % C,
less than 0.01 wt. % B,
less than 0.01 wt. % P,
less than 0.005 wt. % S,
less than 0.05 wt. % Mn,
less than 0.05 wt. % Si, from 0.0001 to 0.05 wt. % O,
from 0.0001 to 0.01 wt. % N,
less than 0.01 wt. % O in the form of a magnesium oxide,
less than 0.01 wt. % O in the form of an aluminium oxide,
less than 0.01 wt. % O in the form of a cerium oxide,
less than 0.01 wt. % O in the form of a calcium oxide, and
less than 0.01 wt. % O in the form of a chromium oxide,
the Cr, Ni, Mo and Co components are major constituents of the second part (30) with at least 95 wt. % of the alloy being Cr, Ni, Mo and Co.

2. Composite wire (10) according to the preceding claim, wherein the composite wire (10) further comprises a core part (40), which is at least partially surrounded by the second part (30) when seen in a cross section.

3. Composite wire (10) according to the preceding claim, wherein the core part (40) is a metallic component.

4. Composite wire (10) according to claim 1, wherein the second part (30) at least partially surrounds the first part (20) when seen in a cross section.

5. Composite wire (10) according to the preceding claim, wherein the composite wire (10) further comprises a circumference part (80), which at least partially surrounds the second part (30) when seen in a cross section, and wherein the circumference part is preferably biocompatible.

6. Composite wire (10) according to one of the preceding claims, wherein a diameter of the composite wire (10) is in a range of 10 to 500 $\mu$m.

7. Composite wire (10) according to one of the preceding claims, wherein the composite wire (10) further comprises a coating as outermost part at least partially surrounding all other parts.

8. Composite wire (10) according to the preceding claim, wherein the coating provides electrical insulation.

9. Composite wire (10) according to claim 7, wherein the coating comprises at least one of a group of enzymes, antimicrobials, and steroids.

10. A coil comprising at least two composite wires (10) according to one of the preceding claims wound together.

11. Coil according to the preceding claim, wherein at least some of the composite wires (10) are electrically insulated from each other.

12. A cable (100) comprising at least three composite wires (10) according to one of the claims 1 to 9 stranded together.

13. A medical device (50) comprising at least one of the group of a composite wire (10), a coil and a cable (100) according to one of the preceding claims as a lead (140).

14. Medical device (50) according to the preceding claim, wherein the lead is configured for at least one of a group of: pacing, defibrillating, cardiac resynchronization, and stimulation to nerves or neural tissue in deep brain, spinal cord, vagus nerve or a peripheral nerve.

**15.** A manufacturing method for a composite wire (10), comprising the following steps:

- providing a first part (20) and a second part (30) concentric to each other when seen in a cross section, and
- joining the first part (20) and the second part (30), which is forming an outer shell for the first part (20), together,

wherein the first part (20) is a metallic component, and

wherein the second part (30) comprises an alloy consisting of the following alloy components:

    a) Cr in the range from 10 to 30 wt. %;
    b) Ni in the range from 20 to 50 wt. %;
    c) Mo in the range from 2 to 20 wt. %; and
    d) Co in the range from 10 to 50 wt. %;

wherein the Al content of the alloy is less than 0.01 wt. %;
wherein each wt. % is based on the total weight of the alloy,
wherein the alloy further contains
less than 0.005 wt. % Mg,
less than 0.005 wt. % Ca,
less than 0.005 wt. % Ce,
less than 0.1 wt. % Ti, from 0.0001 to 1 wt. % Fe,
less than 0.1 wt. % C,
less than 0.01 wt. % B,
less than 0.01 wt. % P,
less than 0.005 wt. % S,
less than 0.05 wt. % Mn,
less than 0.05 wt. % Si,
from 0.0001 to 0.05 wt. % O,
from 0.0001 to 0.01 wt. % N,
less than 0.01 wt. % O in the form of a magnesium oxide,
less than 0.01 wt. % O in the form of an aluminium oxide,
less than 0.01 wt. % O in the form of a cerium oxide,
less than 0.01 wt. % O in the form of a calcium oxide, and
less than 0.01 wt. % O in the form of a chromium oxide,
the Cr, Ni, Mo and Co components are major constituents of the second part (30) with at least 95 wt. % of the alloy being Cr, Ni, Mo and Co.

**Patentansprüche**

**1.** Verbunddraht (10), umfassend

- einen ersten Teil (20), und
- einen zweiten Teil (30), der eine Außenhülle für den ersten Teil (20) bildet,

wobei der erste Teil (20) eine metallische Komponente ist und

wobei der zweite Teil (30) eine Legierung umfasst, die aus den folgenden Legierungskomponenten besteht:

    a) Cr im Bereich von 10 bis 30 Gew.-%;
    b) Ni im Bereich von 20 bis 50 Gew.-%;
    c) Mo im Bereich von 2 bis 20 Gew.-%; und
    d) Co im Bereich von 10 bis 50 Gew.-%;

wobei der Al-Gehalt der Legierung weniger als 0,01 Gew.-% beträgt;
wobei jedes Gew.-% auf das Gesamtgewicht der Legierung bezogen ist,
wobei die Legierung ferner enthält
zu weniger als 0,005 Gew.-% Mg,

zu weniger als 0,005 Gew.-% Ca,
zu weniger als 0,005 Gew.-% Ce,
zu weniger als 0,1 Gew.-% Ti,
zu von 0,0001 bis 1 Gew.-% Fe,
zu weniger als 0,1 Gew.-% C,
zu weniger als 0,01 Gew.-% B,
zu weniger als 0,01 Gew.-% P,
zu weniger als 0,005 Gew.-% S,
zu weniger als 0,05 Gew.-% Mn,
zu weniger als 0,05 Gew.-% Si, von 0,0001 bis 0,05 Gew.-% O,
zu von 0,0001 bis 0,01 Gew.-% N,
zu weniger als 0,01 Gew.-% O in Form eines Magnesiumoxids,
zu weniger als 0,01 Gew.-% O in Form eines Aluminiumoxids,
zu weniger als 0,01 Gew.-% O in Form eines Ceroxids,
zu weniger als 0,01 Gew.-% O in Form eines Calciumoxids und
zu weniger als 0,01 Gew.-% O in Form eines Chromoxids,
wobei die Komponenten Cr, Ni, Mo und Co Hauptbestandteile des zweiten Teils (30) sind, wobei mindestens 95 Gew.-% der Legierung Cr, Ni, Mo und Co sind.

2. Verbunddraht (10) nach dem vorstehenden Anspruch, wobei der Verbunddraht (10) ferner einen Kernteil (40) umfasst, der, in einem Querschnitt gesehen, mindestens teilweise von dem zweiten Teil (30) umgeben ist.

3. Verbunddraht (10) nach dem vorstehenden Anspruch, wobei der Kernteil (40) eine metallische Komponente ist.

4. Verbunddraht (10) nach Anspruch 1, wobei der zweite Teil (30) den ersten Teil (20) in einem Querschnitt betrachtet mindestens teilweise umgibt.

5. Verbunddraht (10) nach dem vorstehenden Anspruch, wobei der Verbunddraht (10) femer einen Umfangsteil (80) umfasst, der den zweiten Teil (30), in einem Querschnitt betrachtet, mindestens teilweise umgibt, und wobei der Umfangsteil vorzugsweise biokompatibel ist.

6. Verbunddraht (10) nach einem der vorstehenden Ansprüche, wobei ein Durchmesser des Verbunddrahtes (10) in einem Bereich von 10 bis 500 $\mu$m liegt.

7. Verbunddraht (10) nach einem der vorstehenden Ansprüche, wobei der Verbunddraht (10) ferner eine Beschichtung als äußersten Teil umfasst, die alle anderen Teile mindestens teilweise umgibt.

8. Verbunddraht (10) nach dem vorstehenden Anspruch, wobei die Beschichtung eine elektrische Isolierung bereitstellt.

9. Verbunddraht (10) nach Anspruch 7, wobei die Beschichtung mindestens eines aus einer Gruppe von Enzymen, antimikrobiellen Mitteln und Steroiden umfasst.

10. Spule, umfassend mindestens zwei Verbunddrähte (10) nach einem der vorstehenden Ansprüche, die miteinander verwickelt sind.

11. Spule nach dem vorstehenden Anspruch, wobei mindestens einige der Verbunddrähte (10) elektrisch voneinander isoliert sind.

12. Kabel (100), umfassend mindestens drei Verbunddrähte (10) nach einem der Ansprüche 1 bis 9, die miteinander verseilt sind.

13. Medizinische Vorrichtung (50) umfassend mindestens einen aus der Gruppe eines Verbunddrahtes (10), einer Spule und eines Kabels (100) nach einem der vorstehenden Ansprüche als eine Leitung (140).

14. Medizinische Vorrichtung (50) nach dem vorstehenden Anspruch, wobei die Leitung konfiguriert ist für mindestens eine aus der Gruppe von: Stimulation, Defibrillation, kardiale Resynchronisation und Stimulation von Nerven oder neuralem Gewebe in der Tiefe des Gehirns, des Rückenmarks, des Vagusnervs oder eines peripheren Nervs.

**15.** Herstellungsverfahren für einen Verbunddraht (10), umfassend die folgenden Schritte:

- Bereitstellen eines ersten Teils (20) und eines zweiten Teils (30), die in einem Querschnitt betrachtet konzentrisch zueinander sind, und
- Verbinden des ersten Teils (20) und des zweiten Teils (30), der eine äußere Hülle für den ersten Teil (20) bildet, miteinander,

wobei der erste Teil (20) eine metallische Komponente ist, und

wobei der zweite Teil (30) eine Legierung umfasst, die aus den folgenden Legierungskomponenten besteht:

a) Cr im Bereich von 10 bis 30 Gew.-%;
b) Ni im Bereich von 20 bis 50 Gew.-%;
c) Mo im Bereich von 2 bis 20 Gew.-%; und
d) Co im Bereich von 10 bis 50 Gew.-%;

wobei der Al-Gehalt der Legierung weniger als 0,01 Gew.-% beträgt;
wobei jedes Gew.-% auf das Gesamtgewicht der Legierung bezogen ist,
wobei die Legierung ferner enthält
zu weniger als 0,005 Gew.-% Mg,
zu weniger als 0,005 Gew.-% Ca,
zu weniger als 0,005 Gew.-% Ce,
zu weniger als 0,1 Gew.-% Ti,
zu von 0,0001 bis 1 Gew.-% Fe,
zu weniger als 0,1 Gew.-% C,
zu weniger als 0,01 Gew.-% B,
zu weniger als 0,01 Gew.-% P,
zu weniger als 0,005 Gew.-% S,
zu weniger als 0,05 Gew.-% Mn,
zu weniger als 0,05 Gew.-% Si,
zu von 0,0001 bis 0,05 Gew.-% O,
zu von 0,0001 bis 0,01 Gew.-% N,
zu weniger als 0,01 Gew.-% O in Form eines Magnesiumoxids,
zu weniger als 0,01 Gew.-% O in Form eines Aluminiumoxids,
zu weniger als 0,01 Gew.-% O in Form eines Ceroxids,
zu weniger als 0,01 Gew.-% O in Form eines Calciumoxids und
zu weniger als 0,01 Gew.-% O in Form eines Chromoxids,
wobei die Komponenten Cr, Ni, Mo und Co Hauptbestandteile des zweiten Teils (30) sind, wobei mindestens 95 Gew.-% der Legierung Cr, Ni, Mo und Co sind.

## Revendications

**1.** Fil composite (10), comprenant

- une première partie (20), et
- une deuxième partie (30), formant une enveloppe externe pour la première partie (20),

dans lequel la première partie (20) est un composant métallique, et

dans lequel la deuxième partie (30) comprend un alliage constitué des composants d'alliage suivants :

a) Cr dans la plage allant de 10 à 30 % en poids ;
b) Ni dans la plage allant de 20 à 50 % en poids ;
c) Mo dans la plage allant de 2 à 20 % en poids ; et
d) Co dans la plage allant de 10 à 50 % en poids ;

dans lequel la teneur en Al de l'alliage est inférieure à 0,01 % en poids ;

dans lequel chaque % en poids est basé sur le poids total de l'alliage,
dans lequel l'alliage contient en outre
moins de 0,005 % en poids de Mg,
moins de 0,005 % en poids de Ca,
moins de 0,005 % en poids de Ce,
moins de 0,1 % en poids de Ti,
de 0,0001 à 1 % en poids de Fe,
moins de 0,1 % en poids de C,
moins de 0,01 % en poids de B,
moins de 0,01 % en poids de P,
moins de 0,005 % en poids de S,
moins de 0,05 % en poids de Mn,
moins de 0,05 % en poids de Si, de 0,0001 à 0,05 % en poids de O,
de 0,0001 à 0,01 % en poids de N,
moins de 0,01 % en poids de O sous la forme d'un oxyde de magnésium,
moins de 0,01 % en poids de O sous la forme d'un oxyde d'aluminium,
moins de 0,01 % en poids de O sous la forme d'un oxyde de cérium,
moins de 0,01 % en poids de O sous la forme d'un oxyde de calcium, et
moins de 0,01 % en poids de O sous la forme d'un oxyde de chrome,
les composants Cr, Ni, Mo et Co sont des constituants majeurs de la deuxième partie (30) avec au moins 95 % en poids de l'alliage étant Cr, Ni, Mo et Co.

2. Fil composite (10) selon la revendication précédente, dans lequel le fil composite (10) comprend en outre une partie d'âme (40), qui est au moins partiellement entourée par la deuxième partie (30), vue en coupe transversale.

3. Fil composite (10) selon la revendication précédente, dans lequel la partie d'âme (40) est un composant métallique.

4. Fil composite (10) selon la revendication 1, dans lequel la deuxième partie (30), vue en coupe transversale, entoure au moins partiellement la première partie (20).

5. Fil composite (10) selon la revendication précédente, dans lequel le fil composite (10) comprend en outre une partie de circonférence (80), qui entoure au moins partiellement la deuxième partie (30) vue en coupe transversale, et dans lequel la partie de circonférence est de préférence biocompatible.

6. Fil composite (10) selon l'une des revendications précédentes, dans lequel un diamètre du fil composite (10) est dans une plage de 10 à 500 μm.

7. Fil composite (10) selon l'une des revendications précédentes, le fil composite (10) comprenant en outre un revêtement en tant que partie la plus extérieure entourant au moins partiellement toutes les autres parties.

8. Fil composite (10) selon la revendication précédente, dans lequel le revêtement fournit une isolation électrique.

9. Fil composite (10) selon la revendication 7, dans lequel le revêtement comprend au moins un parmi un groupe d'enzymes, d'agents antimicrobiens, et de stéroïdes.

10. Bobine comprenant au moins deux fils composites (10) selon l'une des revendications précédentes enroulés ensemble.

11. Bobine selon la revendication précédente, dans laquelle au moins certains des fils composites (10) sont électriquement isolés les uns des autres.

12. Câble (100) comprenant au moins trois fils composites (10) selon l'une des revendications 1 à 9 toronnés ensemble.

13. Dispositif médical (50) comprenant au moins un parmi le groupe d'un fil composite (10), d'une bobine et d'un câble (100) selon l'une des revendications précédentes en tant que conducteur (140).

14. Dispositif médical (50) selon la revendication précédente, dans lequel le conducteur est configuré pour au moins un parmi un groupe de : stimulation, défibrillation, resynchronisation cardiaque, et stimulation des nerfs ou du tissu nerveux dans le cerveau profond, la moelle épinière, le nerf vague ou un nerf périphérique.

15. Procédé de fabrication pour un fil composite (10), comprenant les étapes suivantes :

- la fourniture d'une première partie (20) et d'une deuxième partie (30) concentriques l'une à l'autre, vues en coupe transversale, et
- le raccordement de la première partie (20) et de la deuxième partie (30), qui forme une enveloppe externe pour la première partie (20), l'une à l'autre,

dans lequel la première partie (20) est un composant métallique, et

dans lequel la deuxième partie (30) comprend un alliage constitué des composants d'alliage suivants :

a) Cr dans la plage allant de 10 à 30 % en poids ;
b) Ni dans la plage allant de 20 à 50 % en poids ;
c) Mo dans la plage allant de 2 à 20 % en poids ; et
d) Co dans la plage allant de 10 à 50 % en poids ;

dans lequel la teneur en Al de l'alliage est inférieure à 0,01 % en poids ; dans lequel chaque % en poids est basé sur le poids total de l'alliage, dans lequel l'alliage contient en outre
moins de 0,005 % en poids de Mg,
moins de 0,005 % en poids de Ca,
moins de 0,005 % en poids de Ce,
moins de 0,1 % en poids de Ti,
de 0,0001 à 1 % en poids de Fe,
moins de 0,1 % en poids de C,
moins de 0,01 % en poids de B,
moins de 0,01 % en poids de P,
moins de 0,005 % en poids de S,
moins de 0,05 % en poids de Mn,
moins de 0,05 % en poids de Si,
de 0,0001 à 0,05 % en poids de O,
de 0,0001 à 0,01 % en poids de N,
moins de 0,01 % en poids de O sous la forme d'un oxyde de magnésium,
moins de 0,01 % en poids de O sous la forme d'un oxyde d'aluminium,
moins de 0,01 % en poids de O sous la forme d'un oxyde de cérium,
moins de 0,01 % en poids de O sous la forme d'un oxyde de calcium, et
moins de 0,01 % en poids de O sous la forme d'un oxyde de chrome,
les composants Cr, Ni, Mo et Co sont des constituants majeurs de la deuxième partie (30) avec au moins 95 % en poids de l'alliage étant Cr, Ni, Mo et Co.

Fig. 1

Fig. 2

Fig. 3

Fig. 4

Fig. 5

Fig. 6

Fig. 7

Fig. 8

Fig. 9

Fig. 10

Fig. 11

Fig. 12

Example 1 /Ag28% (inventive) vs. Example 2 /Ag28% (comparative): S-N-curve for 0.127 mm wire

Fig. 13

EP 3 415 649 B1

Fig. 14

Fig. 15

Fig. 16

Fig. 17

**REFERENCES CITED IN THE DESCRIPTION**

**Patent documents cited in the description**

- WO 2005026399 A1
- US 20050051243 A1